(19) Europäisches Patentamt
European Patent Office
Office européen des brevets

(11) Numéro de publication: **0 069 033**
**B1**

# (12) FASCICULE DE BREVET EUROPEEN

(45) Date de publication du fascicule du brevet: **04.09.85**

(21) Numéro de dépôt: **82420080.2**

(22) Date de dépôt: **21.06.82**

(51) Int. Cl.[4]: **C 07 D 211/90**, C 07 D 213/82, C 07 D 213/85, A 01 N 43/40, C 07 D 405/12 // C07C69/14, C07C101/28

(54) **Herbicides à fonction amide et ester dérivés de la pyridine ainsi que leur procédé de préparation et leur application.**

(30) Priorité: **25.06.81 FR 8112698**

(43) Date de publication de la demande: **05.01.83 Bulletin 83/1**

(45) Mention de la délivrance du brevet: **04.09.85 Bulletin 85/36**

(84) Etats contractants désignés: **AT BE CH DE FR GB IT LI LU NL SE**

(56) Documents cités:
**EP - A - 0 003 105**
**EP - A - 0 044 262**
**FR - A - 998 225**
**FR - A - 2 248 028**
**FR - A - 2 381 029**
**FR - A - 2 387 960**

**PATENTS ABSTRACTS OF JAPAN, vol.1, no.70, 8 juillet 1977**

(73) Titulaire: **RHONE-POULENC AGROCHIMIE, 14-20, rue Pierre Baizet, F-69009 Lyon (FR)**

(72) Inventeur: **de Reinach Hirtzbach, François, 107, Rue Garibaldi, F-69006 Lyon (FR)**
Inventeur: **Ambrosi, Dominique, 15, Allée des Hautinières, F-69260 Charbonnières-les-Bains (FR)**

(74) Mandataire: **Chaumette, Michel et al, RHONE-POULENC AGROCHIMIE BP 9163-09, F-69263 Lyon Cedex 1 (FR)**

**EP 0 069 033 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne ce nouveaux composés à fonction amice dérivés de la pyridine, ainsi que leur procédé de préparation, les compositions herbicides les contenant et leur application pour le désherbage sélectif de cultures, notamment le coton et le tournesol.

On a déjà proposé (brevet européen no 3105), comme agents de protection des plantes, des dérivés pyridiniques à fonction amide et plus particulièrement le dianilide dérivé de la dicarboxy-3,5 diméthyl-2,6 pyridine.

Toutefois, de tels composés ne sont pas satisfaisants comme herbicides. On a aussi décrit des dérivés de la dihydropyridine (brevet français 998225) à fonction amide ou ester, mais, ces produits sont simplement connus comme agent de blanchiment des articles textiles.

L'invention a pour but de procurer de nouveaux herbicides sélectifs de la famille des dérivés pyridiniques.

Il a maintenant été trouvé que ce but pouvait être atteint grâce aux nouveaux dérivés selon l'invention.

Ces dérivés sont des produits de formule:

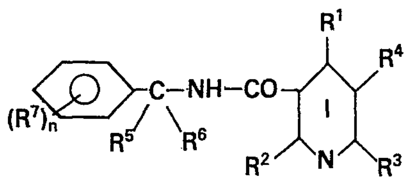

(I)

dans laquelle:

- $R^1$, $R^2$ et $R^3$, identiques ou différents, représentent l'atome d'hydrogène ou un radical alkyle inférieur, ou un radical alkoxy inférieur, ou un radical alkoxyalkyle ayant de 2 à 8 atomes de carbone,
- $R^4$ représente le radical carboxy, éventuellement sous forme sel ou ester ou le radical cyano, ou un radical cyanoalkyle inférieur ou un radical alkoxyalkyle ayant de 2 à 8 atomes de carbone,
- $R^5$ et $R^6$ représentent l'atome d'hydrogène ou un radical alkyle inférieur, ou constituent ensemble un radical unique divalent alkylène ayant de 2 à 5 atomes de carbone, l'un de ces radicaux $R^5$ et $R^6$ pouvant en outre représenter un radical cyano,
- $R^7$ représente un atome d'halogène ou un radical alkyle inférieur, ou un radical alkoxy inférieur ou un radical alcényle inférieur, ou un radical alcényloxy inférieur (ces divers radicaux alkyle, alkoxy, alcényle, alcényloxy étant éventuellement halogénés) ou un radical nitro ou cyano, ou un radical amino ou deux $R^7$ peuvent former ensemble un radical alkylènedioxy comportant de 1 à 4 atomes de carbone,
- n est un nombre entier, positif ou nul, au plus égal à 5 (n = 0 à 5). Lorsque n est supérieur à 1, les différents substituants $R^7$ peuvent être identiqus ou différents.

L'hétérocycle 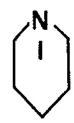 représente un hétérocycle azoté insaturé à 6 côtés pouvant contenir 2 ou 3 insaturations ou doubles liaisons.

Dans le présent exposé, sauf indication particulière contraire, l'adjectif «inférieur», qualifiant un radical organique, signifie que ce radical comporte au plus six atomes de carbone ($C_1$–$C_6$).

Plus spécifiquement l'hétérocycle azoté insaturé

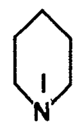

peut représenter soit un noyau pyridinique soit un noyau dihydropyridinique.

Lorsque cet hétérocycle azoté insaturé représente un noyau dihydropyridinique, les produits selon l'invention ont alors pour formule

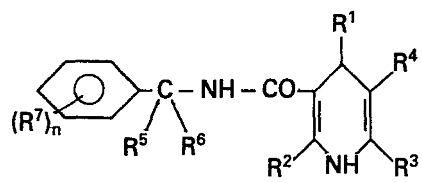

(II)

dans laquelle les divers symboles $R^1$ à $R^7$ et n ont les significations données ci-avant,

Lorsque l'hétérocycle azoté insaturé

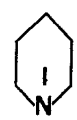

représente un noyau pyridinique, les produits selon l'invention ont alors pour formule:

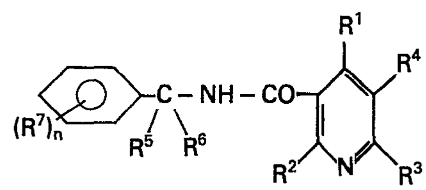

(III)

dans laquelle les divers symboles $R^1$ à $R^7$ et n ont les significations données ci-avant.

L'invention concerne évidemment et également les éventuelles formes tautomères des produits de formule (I).

Da manière avantageuse, le radical $R^4$, lorsque le composé selon l'invention est sous forme sel ou ester, est choisi parmi les radicaux suivants:

– les radicaux COOH salifiés constituant un sel acceptable en agriculture; ce radical COOH peut être salifié par une base minérale (par exemple la soude ou la potasse) ou organique (par exemple une amine primaire, secondaire ou tertiaire, notamment les mono-, di- ou trialkylamines),

– le radical – $COOR^8$, $R^8$ étant un radical alkyle inférieur.

Parmi les divers produits de formule (I), certains constituent une classe préférée; ce sont ceux tels que:

$R^1$ est l'atome d'hydrogène,

$R^2$ et $R^3$ sont indépendemment le radical méthyle, le radical méthoxy, un radical alkoxyalkyle inférieur,

$R^4$ est $COOR^8$, $R^8$ ayant de 1 à 4 atomes de carbone (par exemple les radicaux méthyle et éthyle, en sorte que $R^4$ est alors un carboxylate de méthyle ou d'éthyle), ou un radical cyanoalkyle inférieur ou un radical alkoxyalkyle inférieur,

$R^5$ et $R^6$ sont l'atome d'hydrogène ou, pour l'un d'entre eux, le radical méthyle, ou ils constituent ensemble une chaîne alkylène ayant de 2 à 3 atomes de carbone,

$R^7$ est un atome d'halogène (notamment de chlore ou de fluor), ou un radical alkyle ayant de 1 à 4 atomes de carbone (par exemple méthyle ou éthyle) et éventuellement halogéné (par exemple trifluorméthyle), ou un radical alkoxy ayant de 1 à 4 atomes de carbone,

n est égal à 0, 1 ou 2.

Selon la nature de substituants, les composés de formule (I) peuvent se présenter sous forme racémique ou sous forme d'antipodes optiques. Notamment, lorsque $R^5$ et $R^6$ sont différents, il existe deux formes d'isomères optiques lesquels font partie également de l'invention. Les isomères optiques de même configuration optique que la (1)-diméthyl benzylamine sont avantageux.

Les composés selon l'invention de formule (II) peuvent être préparés selon un procédé qui fait aussi partie de l'invention. Ce procédé consiste à faire réagir ensemble un aldéhyde de formule $R^1$–CHO avec un dérivé aminoéthylénique de formule (IV) et avec un amide cétonique de formule (V):

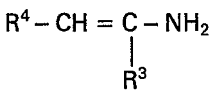

(IV)

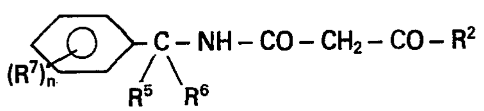

(V)

dans lesquelles $R^1$, $R^2$, $R^3$, $R^4$, $R^5$, $R^6$, $R^7$ et n ont les significations données ci-avant.

La réaction s'effectue selon le schéma réactionnel suivant:

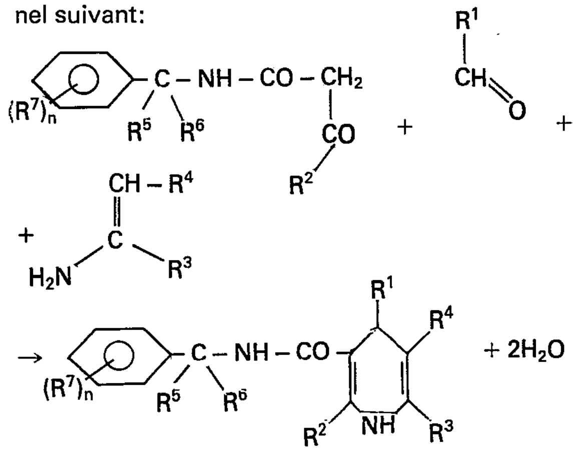

La température réactionnelle est généralement comprise entre +10°C et la température de dégradation des réactifs et/ou produits de réaction. Des températures comprises entre 15 et 100°C conviennent généralement bien. La réaction étant exothermique, on peut opérer initialement à température ambiante, la température s'élevant d'elle-même en cours de réaction; on peut aussi opérer à la température d'ébullition du milieu réactionnel (température de reflux ou solvant lorsqu'on en utilise un).

La réaction du procédé selon l'invention de préparation de produits de formule (II) s'effectue avantageusement en présence d'un solvant organique inerte, c'est-à-dire ne réagissant pas chimiquement avec les réactifs et/ou produits de réaction.

Comme solvants appropriés, on peut citer des solvants organiques usuels, protiques ou aprotiques, comme les hydrocarbures aromatiques, notamment le benzène, le toluène et les xylènes; les hydrocarbures aliphatiques ou cycloaliphatiques notamment l'octane, le décane, le dodécane, le cyclohexane, le méthylcyclohexane; les hydrocarbures halogénés, notamment le dichloro-1,2 éthane, le chloroforme, le tétrachlorure de carbone; les alcanols inférieurs, notamment le méthanol, l'éthanol, l'isopropanol, l'alcool tertiobutylique; les éthers notamment l'éther éthylique; les nitriles notamment l'acétonitrile; les amides notamment le diméthylformamide; les cétones notamment l'acétone et la méthylisobutylcétone.

La réaction du procédé selon l'invention de préparation de produits de formule (II) s'effectue le plus commodément par simple mise en contact des différents réactifs en présence du solvant. D'une manière avantageuse, on préfère dissoudre l'amide cétonique de formule (V) et le dérivé aminoéthylénique de formule (IV) dans le solvant choisi, puis l'on fait réagir l'aldéhyde $R^1CHO$ sur la solution ainsi obtenue.

Les différents réactifs sont mis en œuvre en quantité de préférence stoéchiométrique; il est aussi possible d'utiliser des quantités s'écartant jusqu'à 10%, et même 30% (en mole), de la stoéchiométrie.

La concentration en réactifs du milieu réactionnel est habituellement supérieure à 5% en poids et inférieure à la limite de solubilité des réactifs; des concentrations de 10 à 40% en poids conviennent généralement bien.

En fin de réaction, le produit de formule (II) est séparé du mélange réactionnel par les techniques usuelles, le plus souvent par simple cristallisation par refroidissement.

Les isomères optiques des produits selon l'invention peuvent se préparer selon tout procédé connu en soi. Un procédé consiste à séparer les antipodes optiques de mélanges racémiques. Un procédé plus commode consite à utiliser simplement, comme réactif, un amide cétonique de formule (V) optiquement actif, lui-même dérivant d'une amine optiquement active de formule:

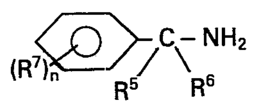

(VI)

La réaction du procédé de l'invention de préparation de produits de formule (II) ne s'accompagne pas d'inversion optique en sorte qu'on choisit avantageusement des amines de formule (VI) de même configuration que celle du produit recherché.

L'aldéhyde $R^1CHO$ et le réactif de formule (IV) sont généralement des produits bien connus, voire même commerciaux. L'amide cétonique de formule (V) peut être préparé par exemple par un procédé tel que décrit dans «Organic Syntheses, Collective Volume 3, page 10» à partir des matières premières appropriées.

Les composés selon l'invention de formule (III) peuvent être préparés selon un procédé qui fait aussi partie de l'invention.

Ce procédé est caractérisé en ce qu'on déshydrogène une dihydropyridine de formule (II).

Cette déshydrogénation peut être effectuée selon des méthodes en soi connues, décrites notamment dans Chemical Reviews 1972, Vol. 72, No 1, p. 31, telles que:

– action d'un oxydant tel que l'acide nitrique, l'acide nitreux (généralement formé in situ par action du nitrite de sodium sur l'acide acétique), l'acide chromique, l'iode ou le soufre ou le permanganate de potassium, ou

– chauffage de la dihydropyridine de formule (II), éventuellement en présence d'un catalyseur de déshydrogénation, tel que, par exemple, le palladium.

Dans le cas de certains des composés préparés, on a observé, de plus, que la déshydrogénation de la dihydropyridine pour donner la pyridine correspondante s'effectue parfois spontanément, à l'air libre ou sous atmosphère contenant de l'oxygène, dès la température ambiante, sans utilisation de catalyseur, au bout d'un temps plus ou moins long (par exemple au cours d'une dissolution dans l'acide acétique entre 20 et 50°C).

Avantageusement, le passage de la dihydropyridine de formule (II) à la pyridine de formule (III) s'effectue par action du nitrite de sodium sur une suspension de la dihydropyridine de formule (II) dans l'acide acétique. La réaction étant exothermique, il est généralement préféré de refroidir le mélange réactionnel de façon à ce que sa température ne dépasse pas 25°C.

La préparation de produits de formule (III) à partir de produits de formule (II) ne s'accompagne pas non plus d'inversion optique en sorte que les produits de formule (II) et les produits de formule (III) qui en dérivent ont même configuration optique.

Les composés répondant à la formule (III), pour lesquels $R_4$ représente un radical cyanoalkyle, les autres substituants pouvant avoir les mêmes significations que dans la formule (III), peuvent être préparés par action du cyanure de sodium sur une chloroalkyle pyridine de formule (VII):

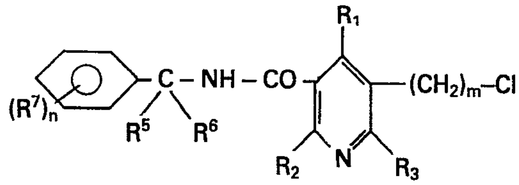

(VII)

dans laquelle m est égal à un nombre de 1 à 4.

La réaction s'effectue avantageusement en milieu aqueux, à température ordinaire.

Les composés répondant à la formule (III) pour lesquels $R_4$ représente un radical alkoxyalkyle, les autres substituants pouvant avoir les mêmes significations que dans la formule (III) peuvent être préparés par action d'un alcanolate de métal alcalin approprié, sur la chloroalkyle-pyridine de formule (VII).

Cette réaction s'effectue avantageusement par un alcanol anhydre, par exemple le méthanol, au moyen du sodium.

Le composé (VII) peut être préparé à partir d'un composé répondant à la formule (III), pour lequel $R_4$ est un radical méthoxycarbonyle en transformant ce groupe méthoxycarbonyle en un groupe hydroxyalkyle (par exemple par réduction au moyen d'un hydrure d'aluminium et lithium), puis en transformant ce groupe hydroxyalkyle en un groupe chloroalkyle, par action d'un agent d'halogénation tel que par exemple $SOCl_2$.

De façon analogue, des composés répondant à la formule (II) pour lesquels $R_4$ représente un radical cyanoalkyle ou alkoxyalkyle, peuvent être préparés à partir ces composés de formule (II) pour lesquels $R_4$ représente le radical méthoxycarbonyle.

Les exemples ci-après, donnés à titre non limitatif, illustrent l'invention et montrent comment elle peut être mise en œuvre.

La structure des composés a été confirmée par spectrométrie infra-rouge et/ou par spectrométrie de résonance magnétique nucléaire (RMN); les spectres RMN ont été réalisés à 60 mégahertz dans le diméthylformamide avec l'hexaméthyldisiloxane comme étalon de référence.

Les exemples 1 à 7 illustrent la préparation des composés selon l'invention.

Dans ces exemples $[\alpha]_D^{20}$ désigne le pouvoir rotatoire à 20°C pour la raie D du sodium, la mesure étant effectuée dans le diméthylformamide.

Les exemples 8 à 10 illustrent les compositions herbicides mettant en œuvre les composés selon l'invention, ainsi que l'application de ces compositions.

Les tableaux (I) à (V) donnet la nature et les propriétés physiques des composés préparés; les tableaux (VI) à (XII) illustrent l'application herbicide de ces composés.

Exemple 1:

Préparation de la (d)dihydro-1,4 N-(α-méthylbenzyl)carbamoyl-3 méthoxycarbonyl-5 lutidine-2,6 (composé dextrogyre no 21) de formule:

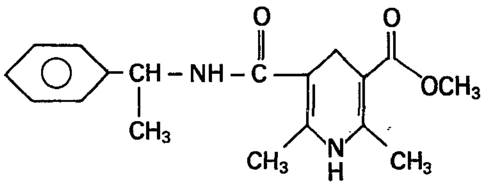

Dans un ballon tricol de 1 litre muni d'un réfrigérant, d'un thermomètre et d'une agitation mécanique centrale, on charge:

– 112 g (0,546 mole) de (1)Nα-méthylbenzyl acétoacétamide,

– 69 g (0,6 mole) de β-aminocrotonate de méthyle,

– 110 ml d'éthanol.

Le mélange est agité à température ambiante puis chauffé jusqu'à 35°C. Lorsque les réactifs sont dissous, on coule dans le mélange 60 ml d'une solution aqueuse à 30% en poids de formaldéhyde (soit environ 0,6 mole). La réaction est exothermique et la température s'élève spontanément jusqu'à 75°C. Le mélange réactionnel est ensuite chauffé à reflux pendant 60 mn.

Après refroidissement, on filtre le précipité. On obtient un mélange contenant le produit cherché et d'autre part, comme sous-produit, la dihydro-1,4 diméthoxycarbonyl-3,5 lutidine-2,6 dont la formation résulte de la condensation de 2 moles de β-aminocrotonate de méthyle et d'une mole de formaldéhyde.

Par recristallisation dans l'éthanol (5 ml/g), on obtient 22 g du produit attendu.

– Rendement par rapport au (1) N-(α-méthylbenzyl) acétoacétamide de départ: 13%

– Point de fusion: 180°C

– Formule brute: $C_{18}H_{22}N_2O_3$

$[\alpha]_D^{20}$: 128°C

Le (1) N-(α-méthylbenzyl) acétoacétamide de départ a été préparé par action de la (1) α-méthylbenzylamine sur le dicétène selon la méthode décrite dans «Organic Syntheses, Collective, Volume 3, page 10» pour la préparation de l'acétoacétanilide.

Exemple 2:

Préparation des composés 1 à 31.

En opérant selon le procédé décrit à l'exemple 1, à partir des matières premières appropriées, les composés no 1 à 31 ont été préparés. Les formules, le point de fusion et le rendement obtenus au cours de la préparation sont indiqués dans les tableaux (I), (II) et (III).

Les produits du tableau (I) ainsi que le composé 31 du tableau (III) ne peuvent pas donner naissance à isomérie optique. Les composés no 20 et 24 à 30 sont des racémiques de même que l'amine utilisée comme réactif de départ. Le composé 21 est optiquement actif comme déjà décrit à l'exemple 1. Le composé 22 est optiquement actif, ayant été préparé à partir d'une amine de départ sous forme d'un isomére optique pur et lévogyre. Le composé no 23 est optiquement actif, ayant été préparé à partir d'une amine de dé- part sous forme d'un isomère optique pur et dextrogyre.

Exemple 3:

Préparation de la (d) méthoxy carbonyl-3 [N-(α-méthyl benzyl)carbamoyl]-5 lutidine-2,6 (composé no 70) de formule:

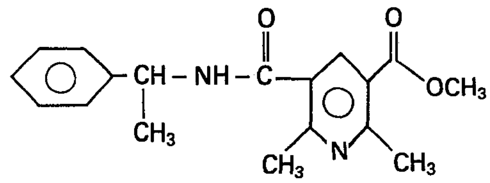

dans un ballon tricol de 250 ml muni d'un réfrigérant, d'un thermomètre et d'une agitation mécanique centrale, ou charge:

– 5 g (0,015 mole) de (d) dihydro-1,4 méthoxy carbonyl-3 [N-(α-méthyl benzyl)carbamoyl]-5 lutidine-2,6 (composé no 21)

– 40 ml d'acide acétique.

Le mélange réactionnel est refroidi à 16°C et on ajoute par petites portions 1,1 g (0,015 mole) de nitrite de sodium.

La réaction exothermique est contrôlée par refroidissement à l'aide d'un bain de glace, de manière à ce que la température du mélange réactionnel n'excède pas 25°C.

Après 30 mn d'agitation vers 20–25°C, le mélange est versé sur de la glace et neutralisé par 50 ml d'ammoniaque concentré. On extrait par deux fois, à l'aide de 100 ml de chlorure de méthylène chaque fois. Après sèchage sur sulfate de sodium et évaporation de la phase organique, on obtient 5 g de produit brut. On filtre sur 150 g de silice et on fait une élution à l'aide d'un mélange chlorure de méthylène-acétone en proportions volumiques respectives 85/15. On obtient ainsi 3,2 g de produit qui cristallise spontanément. On le lave à l'éther et obtient 2,3 g du composé cherché sous forme d'une poudre blanche.

– point de fusion: 129°C

– rendement (à partir de la dihydro lutidine): 46%

– formule brute $C_{18}H_{20}N_2O_3$

– $[\alpha]_D^{20} = +30°$

Exemple 4:

En opérant selon le procédé décrit à l'exemple 3 à partir de matières premières appropriées, les composés no 51 à 87 ont été préparés. Les formules, le point de fusion et le rendement obtenu au cours de la préparation sont indiqués dans les tableaux (IV) et (V).

Les produits des tableaux (IV) et (V) dans lesquels les radicaux $R^5$ et $R^6$ sont identiques ne peuvent pas donner naissance à isomère optique; pour les autres produits, l'amine réactif de départ ainsi que les composés obtenus étaient tous racémiques sauf pour les composés 59, 60 et 70 qui sont des isomères optiques, de même que l'amine réactif de départ, laquelle était, respectivement pour ces trois composés dextrogyre, lévogyre, lévogyre. Le pouvoir rotatoire $[\alpha]_D^{20}$ des composés 59, 60 et 70 est respectivement de −16°, +18°, +30°.

Exemple 5:

Préparation de la méthyle-2 [N-(α-méthylbenzyl)carbamoyl]-3 éthoxycarbonyl-5 méthoxyméthyl-6 pyridine (composé no 88) de formule:

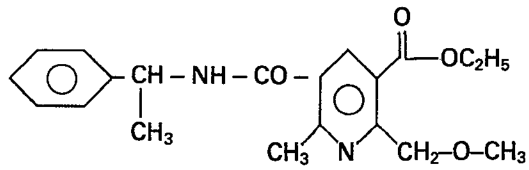

Dans un ballon tricol de 250 ml, on charge 3,6 g de l'amino-3 méthoxy-4 crotonate d'éthylène, 6 g de (S) N-(α-méthylbenzyl) acétoacétamide et 50 ml d'éthanol. Après dissolution des réactifs, on ajoute 4 ml d'une solution aqueuse à 30% (poids/volume) de formaldéhyde, soit environ 40 mM. La réaction est terminée après une heure de reflux à environ 80°C. Après refroidissement, le milieu réactionnel est concentré puis dissous dans 200 ml d'acétone. On l'oxyde par addition d'une solution aqueuse de 4 g de permanganate de potassium (25 mM) dans 100 ml d'eau.

Lorsque la réaction est terminée, le bioxyde de manganèse formé est filtré, rincé au méthanol. La phase organique est concentrée puis filtrée sur 150 g de silice eluée par un mélange égal d'acétate d'éthyle et d'hexane.

On obtient ainsi 32 g du produit attendu sous forme d'une huile visqueuse jaune.

Rendement: 38%.

L'amino-3 méthoxy-4 crotonate d'éthyle utilisé comme produit de départ a été préparé par action de l'ammoniac concentré sur le méthoxyacétate d'éthyle, dans l'éthanol.

Le méthoxyacétate d'éthyle a été préparé comme indiqué ci-après:

Dans un ballon tricol de 1 litre muni d'une agitation mécanique, d'un thermomètre, d'un réfrigérant et d'une ampoule à addition, on place 47 g de malonate d'éthyle et de tertiobutyle préparé selon Organic Synthese Coll. Vol. IV, p. 417. On y ajoute 350 ml d'éther éthylique puis 28,5 g (0,25 mole) d'éthylate de magnésium. Après une heure d'agitation à reflux, le chlorure de méthoxyacéthyle est additionné lentement en refroidissant le milieu réactionnel à une température d'environ 20°C. Après la fin de l'addition, le milieu est chauffé à reflux pendant 1 heure.

Après refroidissement à 10°C, on hydrolyse par 150 ml d'acide sulfurique 2 N. La phase organique est décantée, lavée au bicarbonate de sodium, à l'eau, à l'eau salée puis séchée sur sulfate de sodium. Après concentration, l'huile obtenue est dissoute dans 500 ml de toluène. On y ajoute 1 g d'acide paratoluène sulfonique et chauffe à reflux jusqu'à la fin du dégagement d'isobutène et de gaz carbonique. Après refroidissement, lavage au bicarbonate de sodium, séchage sur sulfate de sodium, la phase organique est concentrée puis distillée sous vide de la trompe à eau.

On obtient 12 g du composé no 88.

Rendement: 30%.

Exemple 6:

Préparation du S [N-(α-méthylbenzyl)carbamoyl]-3 cyanométhyl-5 diméthyl-2,6 pyridine (composé no 89) de formule:

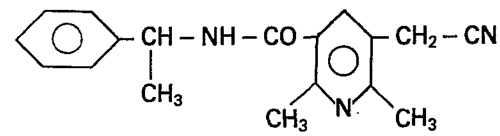

Cette préparation se fait en plusieurs étapes à partir du S [N-α-méthylbenzyl)carbamoyl-]-3 méthoxycarbonyl-5 diméthyl-2,6 pyridine, décrite plus haut en tant que composé no 78.

Dans un ballon tricol avec agitation mécanique centrale, réfrigérant, thermomètre et ampoule à addition, on charge 500 ml de tétrahydrofuranne anhydre, puis 14 g d'hydrure de lithium et aluminium. Après refroidissement entre 5 et 10°C on coule lentement 50 g du composé no 78 dans 300 ml de tétrahydrofuranne. Après la fin de l'addition, le milieu réactionnel est agité 45 mn à 25–30°C. Après refroidissement, le milieu réactionnel est hydrolysé par 14 ml d'eau et 14 ml d'une solution aqueuse de soude à 15% et enfin 42 ml d'eau. La phase organique est séchée sur sulfate de sodium, puis filtrée, on obtient ainsi 45 g de S [N-(α-méthylbenzyl)carbamoyl]-3 hydroxyméthyl-5 diméthyl-2,6 pyridine, fondant à 141°C.

Dans un ballon tricol avec ampoule à addition, agitation mécanique centrale, réfrigérant et thermomètre, on charge 45 g de l'hydroxyméthyl-5 pyridine obtenue précédemment dans un litre de chlorure de méthylène. On y coule 32 g de chlorure de thionyle et on chauffe 30 mn à reflux.

Après refroidissement on neutralise par 170 ml de soude 4 N. Après décantation, séchage, concentration de la phase organique, on obtient, après purification, 37,6 g de S [N-(α-méthylbenzyl)carbamoyl]-3 chlorométhyl-5 diméthyl-2,6 pyridine, fondant à 165°C.

Dans un Erlenmeyer de 1 litre, on dissout à chaud (50°C) 12 g de la chlorométhyl-5 pyridine obtenue précédemment, dans 500 ml d'éthanol.

Puis on coule une solution de 5,8 g de cyanure de sodium dans 100 ml d'eau.

Après 24 h à 50°C, on concentre sous vide, puis ajoute 300 ml de chlorure de méthylène et 100 ml d'eau. La phase organique est décantée, séchée sur sulfate de sodium et concentrée. Le produit obtenu est filtré sur 150 g de silice éluée par le mélange chlorure de méthylène/éther éthylique/méthanol (10-10-l). On obtient ainsi 10,2 g de S [N-(α-méthylbenzyl)carbamoyl]-3 cyanométhyl-5 diméthyl-2,6 pyridine (composé no 89), sous forme de cristaux blancs fondant à 146°C. Rendement 82% (à partir de la chlorométhyl-5 pyridine).

Exemple 7:

Préparation du S [N-(α-méthylbenzyl)carbamoyl]-3 méthoxyméthyl-5 diméthyl-2,6 pyridine (composé no 90), de formule:

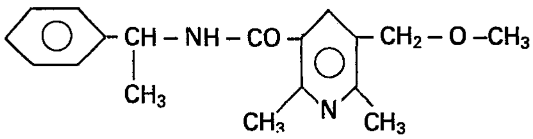

Dans un ballon de 250 ml, on charge 2,1 g du S [N-(α-méthylbenzyl)carbamoyl]-3 chlorométhyl-5 diméthyl-2,6 pyridine dont la préparation est décrite dans l'exemple 6, dans 50 ml de méthanol anhydre. Après dissolution, on y coule une solution de 500 mg de sodium dans 50 ml de méthanol.

Après 2 heures à reflux, la réaction est terminée. On concentre puis redissout dans 200 ml de chlorure de méthylène et 100 ml d'eau. La phase organique est décantée, séchée et concentrée. On obtient ainsi 1,8 g du produit cherché fondant à 125°C.

Exemple 8:

En opérant selon la méthode de l'exemple 7, à partir des matières premières appropriées, on a préparé le S [N-(α-méthylbenzyl)carbamoyl]-3 éthoxyméthyl-5 diméthyl-2,6 pyridine (composé no 91) fondant à 130°C.

Exemple 9:

Application herbicide, en prélevée des espèces végétales.

Dans des pots de 9×9×9 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur.

Après humidification de la terre, les pots sont traités par pulvérisation de bouillie en quantité correspondant à une dose d'application de 500 l/ha et contenant la matière active à la concentration considérée.

La bouillie a été préparée en diluant par une quantité d'eau déterminée, de façon à obtenir la concentration voulue, une poudre mouillable ayant la composition pondérale suivante:

| | |
|---|---|
| – matière active à tester | 20% |
| – support inerte solide: kaolinite | 69% |
| – agent tensioactif (défloculant): lignosulfonate de calcium | 5% |
| – agent tensioactif (mouillant): isopropylnaphtalène sulfonate de sodium | 1% |
| – silice antimottante | 5% |

Cette poudre mouillable a été obtenue en mélangeant et broyant les ingrédients dans un microniseur, de façon à obtenir une grosseur moyenne de particules inférieure à 40 microns.

Selon la concentration en matière active de la bouillie, la dose de matière active appliquée a été de 0,5 à 8 kg/ha.

Les pots traités sont ensuite placés dans des bacs destinés à recevoir l'eau d'arrosage, en subirrigation, et maintenus pendant 35 jours à température ambiante soud 70% d'humidité relative.

Au bout de 35 jours, on compte le nombre de plantes vivantes dans les pots traités par la bouillie contenant la matière active à tester et le nombre de plantes vivantes dans un pot témoin traité selon les mêmes conditions, mais au moyen d'une bouillie ne contenant pas de matière active. On détermine ainsi le pourcentage de destruction des plantes traitées par rapport au témoin non traité. Un pourcentage de destruction égal à 100% indique qu'il y a eu destruction complète de l'espèce végétale considérée et un pourcentage de 0% indique que le nombre de plantes vivantes dans le pot traité est identique à celui dans le pot témoin.

Pour les essais de cet exemple, les espèces végétales utilisées ont été les suivantes:

| | Abrévitation utilisée |
|---|---|
| Adventices monocotylédones: | |
| Folle avoine (Avena fatua) | FAV |
| Digitaire (Digitaria sanguinalis) | DIG |
| Panisse (Echinochloa crus-galli) | PAN |
| Ray-grass (Lolium multiflorum) | RAY |
| Sétaire (Setaria faberii) | SET |
| Vulpin (Alopecurus myosuroides) | VUL |
| Adventices dicotylédones: | |
| Chénopode (Chenopodium sp) | CHE |
| Chrysanthème (Chrysanthemum segetum) | CHR |
| Morelle (Solanum nigrum) | MOR |
| Moutarde (Sinapis arvensis) | MOU |
| Stellaire (Stellaria media) | STE |
| Cultures monocotylédones: | |
| Blé (Triticum sativum) | BLE |
| Maïs (Zea mays) | MAI |
| Orge (Hordeum vulgare) | ORG |
| Riz (Oryza setiv) | RIZ |
| Cultures dicotylédone: | |
| Colza (Brassica oleracea) | COL |
| Coton (Gossypium barbadense) | COT |
| Haricot (Phaseolus vulgaris) | HAR |
| Soja (Glycine max) | SOJ |
| Tournesol (Helianthus annuus) | TOU |

Les résultats observés sont indiqués dans les tableaux de la manière suivante (application en prélevée)

Tableau (VI) – adventices – produits de formule (II)

Tableau (VII) – cultures – produits de formule (II)

Tableau (VIII) – adventices – produits de formule (III)

Tableau (IX) – cultures – produits de formule (III)

Exemple 10:

Application herbicide, en postlevée des espèces végétales.

Dans des pots de 9×9×9 cm remplis de terre agricole légère, on sème un nombre de graines déterminé en fonction de l'espèce végétale et de la grosseur de la graine.

On recouvre ensuite les graines d'un couche de terre d'environ 3 mm d'épaisseur et on laisse germer la graine jusqu'à ce qu'elle donne naissance à une plantule de 5 à 10 cm de hauteur.

Les pots sont alors traités par pulvérisation de bouillie en quantité correspondant à une dose d'application de 500 l/ha et contenant la matière active à la concentration considérée.

La bouillie a été préparée de la même manière qu'à l'exemple 9.

Selon la concentration en matière active de la bouillie, la dose de matière active appliquée a été de 2 à 8 kg/ha.

Les pots traités sont ensuite placés dans des bacs destinés à recevoir l'eau d'arrosage, en subirrigation, et maintenus pendant 30 jours à température ambiante sous 70% d'humidité relative.

Au bout de 30 jours, on compte le nombre de plantes vivantes dans les pots traités par la bouillie contenant la matière active à tester et le nombre de plantes vivantes dans un pot témoin traité selon les mêmes conditions, mais au moyen d'une bouillie ne contenant pas de matière active. On détermine ainsi le pourcentage de destruction des plantes traitées par rapport au témoin non traité. Un pourcentage de destruction égal à 100% indique qu'il y a eu destruction complète de l'espèce végétale considérée et un pourcentage de 0% indique que le nombre de plantes vivantes dans le pot traité est identique à celui dans le pot témoin.

Le nom et l'abrévitation des espèces végétales utilisées sont tels qu'indiqués précédemment.

Les résultats observés sont indiqués dans les tableaux (X) et (XII) pour les produits de formule (II) et dans les tableaux (XI) et (XII) pour les produits de formule (III).

Les résultats obtenus dans les exemples ci-dessus et rapportés aux tableaux (VI) à (XII) montrent l'excellente activité herbicide des composés selon l'invention sur la plupart des adventices traitées, tant graminées que dicotylédones ainsi que leur sélectivité vis-à-vis des cultures considérées.

Pour leur emploi pratique, les composés selon l'invention sont rarement utilisés seuls. Le plus souvent ils font partie de compositions. Ces compositions, utilisables comme agents herbicides, contiennent comme matière active un composé selon l'invention tel que décrit précédemment en association avec les supports solides ou liquides, acceptables en agriculture et les agents tensioactifs également acceptables en agriculture. En particulier sont utilisables les supports inertes et usuels et les agents tensio-actifs usuels.

Ces compositions peuvent contenir aussi toute sorte d'autres ingrédients tels que, par exemple, des colloïdes protecteurs, des adhésifs, des épaissisants, des agents thixotropes, des agents de pénétration, des stabilisants ou des séquestrants ainsi que d'autres matières actives connues à propriétés presticides (notamment insecticides, fongicides ou herbicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Les doses d'emploi des composés selon l'invention peuvent varier dans de larges limites, notamment selon la nature des adventices à éliminer et le degré d'infestation habituel des cultures pour ces adventices.

D'une façon générale, les compositions selon l'invention contiennent habituellement de 0,05 à 95% environ (en poids) d'un ou plusieurs composés selon l'invention, de 1 à 94,95% environ de un ou plusieurs supports solides ou liquides et éventuellement de 0,1 à 20% environ de un ou plusieurs agents tensioactifs.

Selon de qui a déjà été dit les composés selon l'invention sont généralement associés à des supports et éventuellement des agents tensioactifs.

Par le terme «support», dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (agriles, silicates naturels ou synthétiques, silice, résines, cires, engrais solides) ou liquide (eau, alcools, cétones, fractions de pétrole, hydrocarbures aromatiques ou paraffiniques, hydrocarbures chlorés, gaz liquéfiés).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des mines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc génralement sous forme de compositions; ces compositions selon l'invention sont elles-mêmes sous des formes assez diverses, solides ou liquides.

Comme formes de compositions solides, on peut citer les poudres pour poudrage ou dispersion (à teneur en composé de formule [I] pouvant aller jusqu'à 100%) et les granulés, notamment ceux obtenus par extrusion, par compactage, par imprégnation d'un support granulé, par granulation à partir d'une poudre (la teneur en composé de formule [I] dans ces granulés étant entre 0,5 et 80% pour ces derniers cas).

Comme formes de compositions liquides ou destinées à constituer des compositions liquides lors de l'application, on peut citer les suspensions

concentrées, les poudres mouillables (ou poudre à pulvériser) et les pâtes.

Les supsensions concentrées, qui sont applicables en pulvérisation, sont préparées de manière à obtenir un produit fluide stable ne se déposant pas et elles contiennent habituellement de 10 à 75% de matière active, de 0,5 à 15% d'agents tensioactifs, de 0,1 à 10% d'agents thixo- tropes, de 0 à 10% d'additifs appropriés, comme des antimousses, des inhibiteurs de corrosion, des stabilisants, des agents de pénétration et des adhésifs et, comme support, de l'eau ou un liquide organique dans lequel la matière active est peu soluble ou non soluble: certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95% de matière active, et elles contiennent habituellement, en plus du support solide, de 0 à 5% d'un agent mouillant, de 3 à 10% d'un agent dispersant, et, quand c'est nécessaire, de 0 à 10% d'un ou plusieurs stabilisants et/ou autres additifs, comme des agents de pénétration, des adhésifs, ou des agents antimottants ou des colorants.

A titre d'exemple, voici diverses compositions de poudres mouillables:

| | |
|---|---|
| – matière active | 50% |
| – lignosulfonate de calcium (défloculant) | 5% |
| – isopropylnaphtalène sulfonate (agent mouillant anionique) | 1% |
| – silice antimottante | 5% |
| – kaolin (charge) | 39% |

Un autre exemple de poudre mouillable à 80% est donné ci-après:

| | |
|---|---|
| • matière active | 80% |
| • alkylnaphtalène sulfonate de sodium | 2% |
| • lignosulfonate de sodium | 2% |
| • silice antimottante | 3% |
| • kaolin | 13% |

Un autre exemple de poudre mouillable est donné ci-après:

| | |
|---|---|
| • matière active | 50% |
| • alkylnaphtalène sulfonate de sodium | 2% |
| • méthyl cellulose de faible viscosité | 2% |
| • terre de diatomées | 46% |

Un autre exemple de poudre mouillable est donné ci-après:

| | |
|---|---|
| • matière active | 90 % |
| • dioctylsulfosuccinate de sodium | 0,2% |
| • silice synthétique | 9,8% |

Une autre composition de poudre à pulvériser à 70% utilise les constituants suivants:

| | |
|---|---|
| – matière active | 700 g |
| – dibutylnaphtylsulfonate de sodium | 50 g |
| – produit de condensation en proportions 3/2/1 d'acide naphtalène sulfonique, d'acide phénolsulfonique et de formaldéhyde | 30 g |
| – kaolin | 100 g |
| – craie de champagne | 120 g |

Une autre composition de poudre à pulvériser à 40% utilise les constituants suivants:

| | |
|---|---|
| – matière active | 400 g |
| – lignosulfonate de sodium | 50 g |
| – dibutylnaphtalène sulfonate de sodium | 10 g |
| – silice | 540 g |

Une autre composition de poudre à pulvériser à 25% utilise les constituants suivants:

| | |
|---|---|
| – matière active | 250 g |
| – lignosulfonate de calcium | 45 g |
| – mélange équipondéral de craie de champagne et d'hydroxyéthylcellulose | 19 g |
| – dibutylnaphtalène sulfonate de sodium | 15 g |
| – silice | 195 g |
| – craie de champagne | 195 g |
| – kaolin | 281 g |

Une autre composition de poudre à pulvériser à 25% utilise les constituants suivants:

| | |
|---|---|
| – matière active | 250 g |
| – isooctylphénoxy-polyoxyéthylène-éthanol | 25 g |
| – mélange équipondéral de craie de champagne et d'hydrocyéthylcellulose | 17 g |
| – aluminosilicate de sodium | 543 g |
| – Kieselguhr | 165 g |

Une autre composition de poudre à pulvériser à 10% utilise les constituants suivants:

| | |
|---|---|
| – matière active | 100 g |
| – mélange de sels de sodium de sulfates d'acides gras saturés | 30 g |
| – produit de condensation d'acide naphtalène sulfonique et de formaldéhyde | 50 g |
| – kaolin | 820 g |

Pour obtenir ces poudres à pulvériser ou poudres mouillables, on mélange intimement les matières actives dans des mélangeurs appropriés avec les substances additonnelles et on broie avec des moulins ou autres broyeurs appropriés. On obtient par là des poudres à pulvériser dont la muillablilité et la mise en suspension sont avantageuses; on peut les mettre en suspension avec de l'eau à toute concentration désirée et cette suspension est utilisable très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions, par exemple les compositions obtenues en diluant à l'aide d'eau une poudre mouillable selon l'invention, sont comprises dans le cadre général de la présente invention. On utilise le terme de bouillie pour désigner les compositions diluées à l'eau telles qu'on les applique aux cultures.

Toutes ces dispersions ou émulsions aqueuses ou bouillies sont applicables aux cultures à désherber par tout moyen convenable, principalement par pulvérisation, à des doses qui sont généralement de l'ordre de 100 à 1200 litres de bouillie à l'hectare.

Les granulés destinés à être disposés sur le sol sont habituellement préparés de manière qu'ils aient des dimensions comprises entre 0,1 et 2 mm et ils peuvent être fabriqués par agglomération ou imprégnation. De préférence, les granulés contiennent 1 à 25% de matière active et 0 à 10% d'additifs comme des stabilisants, des agents de modification à libération lente, des liants et des solvants.

Selon un exemple de composition de granulé, on utilise les constituants suivants:

| | |
|---|---|
| – matière active | 50 g |
| – épichlorhydrine | 2,5 g |
| – éther de cétyle et de polyglycol | 2,5 g |
| – polyéthylène gylcol | 35 g |
| – kaolin (granulométrie:0,3 à 0,8 mm) | 910 g |

Dans ce cas particulier on mélange la matière active avec l'épichlorhydrine et on disperse dans 60 g d'acétone; on ajoute alors le polyéthylène glycol et l'éther de cétyle et de polyglycol. On arrose le kaolin avec la dispersion obtenue et on évapore ensuite l'acétone sous vide.

Comme indiqué plus haut, l'invention concerne également un procédé de désherbage de cultures, notamment le coton, le tournesol, le blé et l'orge, selon lequel on applique sur les plantes et/ou sur le sol de la zone à désherber une quantité efficace d'au moins un des composés selon l'invention. Ceux-ci sont utilisés pratiquement sous forme des compositions herbicides selon l'invention qui ont été décrites ci-avant. Généralement, des quantités de matière active allant de 0,1 à 10 kg/ha donnent de bons résultats, étant entendu que le choix de la quantité de matière active à utiliser est fonction de l'intensité du problème à résoudre, des conditions climatiques et de la culture considérée. Le traitement est en général effectué en prélevée des cultures et adventices, ou en présemis des cultures avec incorporation dans le sol (cette incorporation est donc une opération complémentaire du procédé de traitement de l'invention), bien que dans certains cas, selon le composé utilisé de bons résultats puissent également être obtenus par des traitements de post-levée. D'autres modes de mise en œuvre du procédé de traitement selon l'invention peuvent encore être utilisés: ainsi on peut appliquer la matière active sur le sol, avec ou sans incorporation, avant repiquage d'une culture.

Le procédé de traitement de l'invention s'applique aussi bien dans le cas de cultures annuelles que dans le cas de cultures pérennes; dans ce dernier cas on préfère appliquer les matières actives de l'invention de manière localisée, par exemple entre les rangs des dites cultures.

Tableau (I)

Composés de formule (II). $R^1 = H$; $R^2 = R^3 = -CH_3$; $R^4 = -COOR^8$; $R^5 = R^6 = H$

| Composé no | $R^8$ | 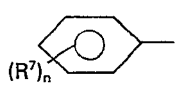 | Point de fusion en °C | Rendement en % |
|---|---|---|---|---|
| 1 | $-CH_3$ | $C_6H_5-$ | 156 | 17 |
| 2 | $-C_2H_5$ | $C_6H_5-$ | 101 | 49 |
| 3 | $-CH_3$ | $p-CH_3-C_6H_4-$ | 186 | 11 |
| 4 | $-C_2H_5$ | $p-CH_3-C_6H_4-$ | 135 | 30 |
| 5 | $-C_2H_5$ | $m-CH_3O-C_6H_4-$ | 119 | 23 |
| 6 | $-C_2H_5$ | $p-CH_3O-C_6H_4-$ | 114 | 22 |
| 7 | $-CH_3$ | $o-Cl-C_6H_4-$ | 128 | 30 |
| 8 | $-C_2H_5$ | $o-Cl-C_6H_4-$ | 134 | 30 |
| 9 | $-CH_3$ | $m-Cl-C_6H_4-$ | 162 | 25 |
| 10 | $-C_2H_5$ | $m-Cl-C_6H_4-$ | 130 | 20 |
| 11 | $-CH_3$ | $p-Cl-C_6H_4-$ | 184 | 23 |
| 12 | $-C_2H_5$ | $p-Cl-C_6H_4-$ | 145 | 28 |
| 13 | $-CH_3$ | $o-F-C_6H_4-$ | 155 | 15 |
| 14 | $-CH_3$ | $p-F-C_6H_4-$ | 178 | 8 |
| 15 | $-C_2H_5$ | $p-F-C_6H_4-$ | 144 | 25 |
| 16 | $-C_2H_5$ | $o,p-Cl_2C_6H_4-$ | 151 | 18 |
| 17 | $-C_2H_5$ | $m,p-Cl_2C_6H_4-$ | 133 | 28 |
| 18 | $-C_2H_5$ | $m-CF_3-C_6H_4-$ | 165 | 11 |
| 19 | $-CH_3$ | 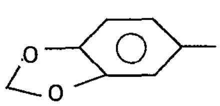 | 157 | 14 |

Tableau (II)

| Composés de formule (II). $R^1 = H$; $R^2 = R^3 = -CH_3$; $R^4 = -COOR^8$; $R^5 = H$; $R^6 = -CH_3$<br>dl, l, d signifient respectivement racémique, lévogyre, dextrogyre | | | | | |
|---|---|---|---|---|---|
| Composé no | $R^8$ | 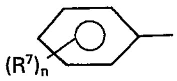 | Isomérie optique de l'amine de départ | Point de fusion en °C | Rendement en % |
| 20 | $-CH_3$ | $C_6H_5-$ | dl | 186 | 19 |
| 21 | $-CH_3$ | $C_6H_5-$ | l | 180 | 13 |
| 22 | $-C_2H_5$ | $C_6H_5-$ | l | 139 | 19 |
| 23 | $-C_2H_5$ | $C_6H_5$ | d | 145 | 10 |
| 24 | $-C_2H_5$ | $C_6H_5-$ | dl | | |
| 25 | $-CH_3$ | $O-CH_3-C_6H_5$ | dl | 145 | 13 |
| 26 | $-C_2H_5$ | $p-Cl-C_6H_4-$ | dl | 163 | 21 |
| 27 | $-C_2H_5$ | $p-F-C_6H_4-$ | dl | 167 | 35 |
| 28 | $-C_2H_5$ | $o,p-Cl_2C_6H_4-$ | dl | 123 | 25 |
| 29 | $-C_2H_5$ | $m,p-Cl_2C_6H_4-$ | dl | 126 | 20 |

Tableau (III)

| Composés de formule (II). $R^1 = H$; $R^2 = R^3 = -CH_3$; $R^4 = -COOR^8$ | | | | |
|---|---|---|---|---|
| Composé no | $R^8$ | 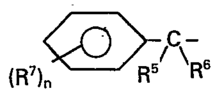 | Point de fusion en °C | Rendement en % |
| 30 | $-C_2H_5$ | 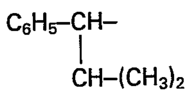 | 100 | 36 |
| 31 | $-C_2H_5$ | 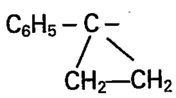 | 139 | 13 |

Tableau (IV)

| Composés de formule (III). $R^1 = H$; $R^2 = R^3 = -CH_3$; $R^4 = -COOR^8$ | | | | | | |
|---|---|---|---|---|---|---|
| Composé no | $R^8$ | $R^5$ | $R^6$ | 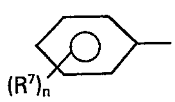 | Point de fusion en °C | Rendement en % lors de la préparation |
| 51 | $-C_2H_5$ | H | H | $o-Cl-C_6H_4-$ | 134 | 80 |
| 52 | $-CH_3$ | H | H | $o-Cl-C_6H_4-$ | 129 | 70 |
| 53 | $-C_2H_5$ | H | H | $m-Cl-C_6H_4-$ | 117 | 60 |
| 54 | $-C_2H_5$ | H | H | $p-F-C_6H_4-$ | 132 | 40 |
| 55 | $-C_2H_5$ | H | $-CH_3$ | $p-Cl-C_6H_4-$ | 150 | 51 |
| 56 | $-C_2H_5$ | H | H | $m-CF_3-C_6H_4-$ | 144 | 28 |
| 57 | $-C_2H_5$ | 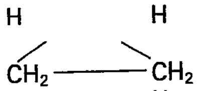 | | $C_6H_5-$ | 118 | 43 |
| 58 | $-C_2H_5$ | H | H | $p-Cl-C_6H_4-$ | 133 | 32 |
| 59 | $-C_2H_5$ | H | $-CH_3$ | $C_6H_5-$ | 90 | 15 |
| 60 | $-C_2H_5$ | $-CH_3$ | H | $C_6H_5-$ | 86 | 32 |
| 61 | $-C_2H_5$ | H | $-CH_3$ | $o-CH_3-C_6H_4-$ | 113 | 63 |
| 62 | $-C_2H_5$ | H | $-CH_3$ | $o,p-Cl_2C_6H_3-$ | 135 | 56 |
| 63 | $-CH_3$ | H | $-CH_3$ | $o-CH_3-C_6H_4-$ | 177 | 89 |
| 64 | $-CH_3$ | H | H | $p-F-C_6H_4$ | 107 | 30 |
| 65 | $-C_2H_5$ | H | $-CH_3$ | $p-F-C_6H_4-$ | 62 | 59 |
| 66 | $-C_2H_5$ | H | $-CH_3$ | $m,p-Cl_2C_6H_3-$ | 132 | 58 |

Tableau (V)

| Composés de formule (III). $R^1 = H$; $R^2 = R^3 = -CH_3$; $R^4 = -COOR^8$ | | | | | | |
|---|---|---|---|---|---|---|
| Composé no | $R^8$ | $R^5$ | $R^6$ | 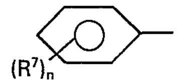 | Point de fusion en °C | Rendement en % lors de la préparation |
| 67 | $-C_2H_5$ | H | H | m,p–$Cl_2C_6H_3$– | 129 | 54 |
| 68 | $-CH_2H_5$ | H | H | p–$CH_3$–$C_6H_4$– | 145 | 47 |
| 69 | $-C_2H_5$ | H | H | o,p–$Cl_2C_6H_3$– | 149 | 62 |
| 70 | $-C_2H_5$ | H | $-CH_3$ | $C_6H_5$– | 129 | 46 |
| 71 | $-C_2H_5$ | H | $-CH_3$ | dichloro–2,5 phényl | 160 | 9 |
| 72 | $-C_2H_5$ | H | H | m,$CH_3O$–$C_6H_4$– | 136 | 56 |
| 73 | $-C_2H_5$ | H | H | p–$CH_3O$–$C_6H_4$– | 132 | 95 |
| 74 | $-C_2H_5$ | $-CH_3$ | $-CH_3$ | $C_6H_5$– | 137 | 35 |
| 75 | $-C_2H_5$ | H | H | $C_6H_5$– | 99 | 53 |
| 76 | $-CH_2H_5$ | H | $-CH_3$ | $C_6H_5$– | 122 | 30 |
| 77 | $-CH_3$ | H | H | $C_6H_5$– | 107 | 50 |
| 78 | $-CH_3$ | H | $-CH_3$ | $C_6H_5$– | 116 | 32 |
| 79 | $-CH_3$ | H | H | p–Cl–$C_6H_4$– | 156 | 42 |
| 80 | $-CH_3$ | H | H | m–Cl–$C_6H_4$– | 165 | 38 |
| 81 | $-CH_3$ | H | H | o–F–$C_6H_4$– | 130 | 32 |
| 82 | $-CH_3$ | H | H | 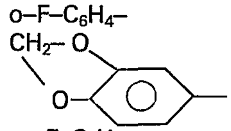 | 152 | 28 |
| 83 | $-CH_3$ | H | H | m–F–$C_6H_4$– | 149 | 40 |
| 84 | $-CH_2H_5$ | H | H | o–$CH_3O$–$C_6H_4$ | 109 | 36 |
| 85 | $-CH_3$ | H | H | $C_6H_5$– | 126 | 24 |
| 86 | $-CH_3$ | H | | m–$NO_2$–$C_6H_4$– | 183 | 43 |
| 87 | $-CH_3$ | H | | p–$CH_3$–$C_6H_4$– | 146 | 34 |

| Composés de formule (III). $R^1 = H$; $R^2 = -CH_3$; $R^5 = H$; $R^6 = CH_3$; n = zéro | | | | |
|---|---|---|---|---|
| Composé no | $R^3$ | $R^4$ | Point de fusion en °C | Rendement en % lors de la préparation |
| 88 | $-CH_2-O-CH_3$ | $-COOC_2H_5$ | huile | 30 |
| 89 | $-CH_3$ | $-CH_2-CN$ | 146 | 88 |
| 90 | $-CH_3$ | $-CH_2-O-CH_3$ | 125 | 86 |
| 91 | $-CH_3$ | $-CH_2-O-C_2H_5$ | 130 | 76 |

Tableau (VI)

| Composé no | Dose kg/ha | Adventices (traitement en) (prélevée) | | | | | | | | | | |
|---|---|---|---|---|---|---|---|---|---|---|---|---|
| | | FAV | DIG | PAN | RAY | SET | VUL | CHE | MOR | MOU | STE | CHR |
| 1 | 8 | 100 | | 100 | 100 | | | 100 | | 100 | | |
| | 2 | 0 | 100 | 50 | 80 | 100 | 90 | 100 | 98 | 85 | 100 | 30 |
| 2 | 8 | 100 | | 100 | 100 | | | 100 | | 100 | | |
| | 2 | 0 | 100 | 0 | 0 | 100 | 10 | 100 | 100 | 100 | 100 | 90 |
| | 0,5 | 0 | 100 | 0 | 0 | 80 | 0 | 98 | 100 | 0 | 90 | 80 |
| 4 | 2 | 20 | 90 | 30 | | | | 100 | | 100 | | |
| 5 | 1 | 20 | | 95 | 80 | | | 100 | | 100 | | |
| 6 | 1 | 20 | | 100 | 90 | | | 100 | | 100 | | |
| 7 | 8 | 100 | | 100 | 100 | | | 100 | | 100 | | |
| | 2 | 0 | 100 | 50 | 90 | 100 | 98 | 100 | 100 | 0 | 100 | 90 |
| 8 | 8 | 95 | | 100 | 100 | | | 100 | | 100 | | |
| | 1 | 0 | 100 | 0 | 0 | 100 | 0 | 100 | 100 | 98 | 100 | 60 |

Tableau (VI) (suite)

| Composé no | Dose kg/ha | Adventices (traitement en) (prélevée) | | | | | | | | | | |
|---|---|---|---|---|---|---|---|---|---|---|---|---|
| | | FAV | DIG | PAN | RAY | SET | VUL | CHE | MOR | MOU | STE | CHR |
| 10 | 8 | 95 | | 100 | 100 | | | 100 | | 100 | | |
| | 2 | 0 | 100 | 80 | 80 | 100 | 60 | 100 | 100 | 20 | 100 | 95 |
| 12 | 8 | 100 | | 100 | 100 | | | 100 | | 100 | | |
| | 1 | 0 | 100 | 50 | 30 | 98 | 30 | 100 | 100 | 0 | 100 | 80 |
| | 0,5 | 0 | 100 | 0 | 0 | 98 | 10 | 100 | 100 | 0 | 100 | 60 |
| 13 | 8 | 70 | | 90 | 100 | | | 100 | | 100 | | |
| 14 | 8 | 100 | | 100 | 100 | | | 100 | | 100 | | |
| 15 | 8 | 100 | | 100 | 100 | | | 100 | | 100 | | |
| | 1 | 0 | 100 | 60 | 60 | 100 | 80 | 100 | 100 | 90 | 100 | 95 |
| 16 | 8 | 70 | | 100 | 98 | | | 100 | | 100 | | |
| | 1 | 0 | 100 | 0 | 0 | 98 | 10 | 100 | 98 | 0 | 100 | 0 |
| 17 | 2 | 20 | | 50 | 30 | | | 100 | | 100 | | |
| | 1 | 0 | 98 | 0 | 0 | 80 | 0 | 100 | 100 | 0 | 100 | 0 |
| 18 | 8 | 100 | | 100 | 100 | | | 100 | | 100 | | |
| 21 | 1 et 8 | 100 | | 100 | 100 | | | 100 | | 100 | | |
| 22 | 8 | 100 | | 100 | 100 | | | 100 | | 100 | | |
| | 0,5 | 10 | 100 | 90 | 40 | 100 | 70 | 100 | 100 | 85 | 100 | 90 |
| 23 | 8 | 20 | | 90 | 20 | | | 100 | | 70 | | |
| | 4 | 0 | 100 | 15 | 0 | 100 | 0 | 100 | 100 | 10 | 60 | 80 |
| 24 | 8 | 100 | | 100 | 100 | | | 100 | | 100 | | |
| | 1 | 5 | 100 | 90 | 80 | 100 | 60 | 100 | 100 | 60 | 100 | 90 |
| 25 | 8 | 80 | | 100 | 100 | | | 100 | | 100 | | |
| 26 | 8 | 100 | | 100 | 100 | | | 100 | | 100 | | |
| | 1 | 0 | 100 | 80 | 50 | 100 | 80 | 100 | 100 | 98 | 100 | 90 |
| 27 | 2 | 90 | | 100 | 95 | | | 100 | | 100 | | |
| 28 | 8 | 90 | | 100 | 70 | | | 100 | | 100 | | |
| | 0,5 | 0 | 95 | 0 | 0 | 95 | 10 | 98 | 100 | 0 | 100 | 0 |
| 29 | 8 | 20 | | 100 | 20 | | | 100 | | 100 | | |
| | 2 | 0 | 100 | 20 | 0 | 98 | 5 | 100 | 100 | 0 | 100 | 0 |
| 30 | 8 | 0 | | 20 | 20 | | | 100 | | 30 | | |
| 31 | 8 | 100 | | 100 | 100 | | | 100 | | 100 | | |
| | 0,5 | 0 | 100 | 20 | 30 | 100 | 20 | 100 | 100 | 70 | 100 | 70 |

Tableau (VII)

| Composé no | Dose kg/ha | Cultures (traitement en) (prélevée) | | | | | | | | |
|---|---|---|---|---|---|---|---|---|---|---|
| | | BLE | MAI | ORG | RIZ | COL | COT | HAR | SOJ | TOU |
| 1 | 8 | | | | | | | 0 | | |
| | 2 | 30 | 5 | 5 | 0 | | 0 | 0 | 20 | 0 |

Tableau (VII) (suite)

| Composé no | Dose kg/ha | Cultures (traitement en) (prélevée) | | | | | | | | |
|---|---|---|---|---|---|---|---|---|---|---|
| | | BLE | MAI | ORG | RIZ | COL | COT | HAR | SOJ | TOU |
| 2 | 8 | | | | | | | 50 | | |
| | 2 | 0 | 0 | 0 | 0 | | 0 | 0 | 0 | 0 |
| | 0,5 | 0 | 0 | 0 | 0 | 10 | 0 | 0 | 0 | 0 |
| 4 | 2 | | | | | | | 0 | | |
| 5 | 1 | | | | | | | 0 | | |
| 6 | 1 | | | | | | | 30 | | |
| 7 | 8 | | | | | | | 0 | | |
| | 2 | 0 | 10 | 0 | 0 | | 0 | 0 | 0 | 0 |
| 8 | 8 | | | | | | | 0 | | |
| | 1 | 0 | 0 | 0 | 0 | | 0 | 0 | 0 | 0 |
| 10 | 8 | | | | | | | 0 | | |
| | 2 | 0 | 10 | 5 | 15 | | 0 | 0 | 0 | 0 |
| 12 | 8 | | | | | | | 100 | | |
| | 1 | 0 | 0 | 10 | 0 | | 0 | 0 | 0 | 0 |
| | 0,5 | 0 | 0 | 0 | 5 | 10 | 0 | 0 | 0 | 0 |
| 13 | 8 | | | | | | | 0 | | |
| 14 | 8 | | | | | | | 100 | | |
| 15 | 8 | | | | | | | 30 | | |
| | 1 | 0 | 0 | 0 | 10 | 20 | 0 | 30 | 5 | 0 |
| 16 | 8 | | | | | | | 0 | | |
| | 1 | 0 | 0 | 0 | 0 | 0 | 0 | 0 | 0 | 0 |
| 17 | 4 | 0 | 0 | 0 | 5 | | 0 | 0 | 20 | 0 |
| | 1 | 0 | 0 | 0 | 0 | 5 | 0 | 0 | 20 | 0 |
| 22 | 8 | | | | | | | | | |
| | 0,5 | 10 | 30 | 0 | 10 | | 0 | 30 | 0 | 0 |
| 23 | 8 | | | | | | | 30 | | |
| | 4 | 0 | 0 | 0 | 0 | | 30 | 0 | 50 | 0 |
| 24 | 8 | | | | | | | 30 | | |
| | 1 | 5 | 10 | 0 | 5 | | 0 | 0 | 20 | 0 |
| 25 | 8 | | | | | | | | | |
| 26 | 1 | 5 | 5 | 0 | 10 | 20 | 0 | 30 | 20 | 0 |
| 27 | 2 | | | | | | | O | | |
| 28 | 0,5 | 0 | 0 | 0 | 30 | 5 | 0 | 0 | 20 | 0 |
| 29 | 2 | 0 | 0 | 0 | 10 | | 0 | 0 | 10 | 0 |
| 30 | 8 | | | | | | | 0 | | |
| 31 | 8 | | | | | | | 0 | | |
| | 0,5 | 0 | 0 | 0 | 0 | | 0 | 0 | 0 | 0 |

Tableau (VIII)

| Composé no | Dose kg/ha | Adventices en prélevée | | | | | | | | | | |
|---|---|---|---|---|---|---|---|---|---|---|---|---|
| | | FAV | DIG | PAN | RAY | SET | VUL | CHE | CHR | MOR | MOU | STE |
| 51 | 8 | 100 | | 100 | 100 | | | 100 | | | 100 | |
| | 2 | 0 | 100 | 20 | 30 | 100 | 40 | 100 | 95 | 100 | 80 | 100 |
| 52 | 8 | 98 | | 100 | 100 | | | 100 | | | 100 | |
| | 1 | 0 | 100 | 50 | 85 | 100 | 95 | 100 | 80 | 100 | 80 | 100 |
| 53 | 8 | 100 | | 100 | 100 | | | 100 | | | 100 | |
| | 1 | 0 | 100 | 60 | 40 | 100 | 60 | 100 | 80 | 100 | 0 | 100 |
| 54 | 8 | 100 | | 100 | 100 | | | 100 | | | 100 | |
| | 0,5 | 0 | 100 | 40 | 0 | 100 | 20 | 100 | 90 | 100 | 80 | 100 |
| 55 | 8 | 100 | | 100 | 100 | | | 100 | | | 100 | |
| | 0,5 | 0 | 100 | 60 | 15 | 100 | 60 | 100 | 20 | 100 | 90 | 100 |
| 56 | 8 | 95 | | 100 | 100 | | | 100 | | | 100 | |
| 57 | 8 | 100 | | 100 | 100 | | | 100 | | | 100 | |
| | 0,5 | 0 | 100 | 20 | 10 | 100 | 30 | 100 | 30 | 100 | 60 | 100 |
| 58 | 8 | 100 | | 100 | 100 | | | 100 | | | 100 | |
| | 2 | 0 | 100 | 90 | 80 | 100 | 70 | 100 | 95 | 100 | 98 | 100 |
| 59 | 8 | 60 | | 100 | 90 | | | 100 | | | 100 | |
| | 2 | 0 | 100 | 30 | 20 | 100 | 0 | 100 | 60 | 100 | 95 | 100 |
| 60 | 8 | 100 | | 100 | 100 | | | 100 | | | 100 | |
| | 0,5 | 10 | 100 | 95 | 30 | 100 | 80 | 100 | 90 | 100 | 30 | 100 |
| 61 | 8 | 95 | | 100 | 100 | | | 100 | | | 100 | |
| 62 | 8 | 60 | | 100 | 80 | | | 100 | | | 100 | |
| | 0,5 | 0 | 98 | 0 | 10 | 100 | 10 | 100 | 0 | 100 | 0 | 100 |
| 63 | 8 | 95 | | 100 | 60 | | | 100 | | | 100 | |
| 64 | 8 | 100 | | 100 | 100 | | | 100 | | | 100 | |
| | 2 | 95 | | 100 | 100 | | | 100 | | | 100 | |
| 65 | 8 | 100 | | 100 | 100 | | | 100 | | | 100 | |
| | 2 | 98 | | 100 | 100 | | | 100 | | | 100 | |
| 66 | 8 | 40 | | 98 | 40 | | | 100 | | | 100 | |
| | 2 | 20 | | 95 | 20 | | | 100 | | | 100 | |
| | 1 | 0 | 98 | 0 | 0 | 98 | 0 | 100 | 0 | 100 | 0 | 100 |
| 67 | 8 | 50 | | 100 | 90 | | | 100 | | | 100 | |
| | 2 | 20 | | 60 | 20 | | | 100 | | | 100 | |
| 68 | 8 | 70 | | 100 | 85 | | | 100 | | | 100 | |
| | 1 | 20 | | 50 | 20 | | | 100 | | | 100 | |
| 69 | 8 | 100 | | 100 | 100 | | | 100 | | | 100 | |
| | 2 | 0 | 98 | 30 | 15 | 100 | 70 | 100 | 0 | 100 | 0 | 100 |
| | 1 | 30 | | 98 | 70 | | | 100 | | | | |

Tableau (VIII) (suite)

| Composé no | Dose kg/ha | Adventices en prélevée | | | | | | | | | | |
|---|---|---|---|---|---|---|---|---|---|---|---|---|
| | | FAV | DIG | PAN | RAY | SET | VUL | CHE | CHR | MOR | MOU | STE |
| 70 | 8 | 100 | | 100 | 100 | | | 100 | | | 100 | |
| | 1 | 60 | | 98 | 100 | | | 100 | | | 100 | |
| 71 | 8 | 10 | | 90 | 10 | | | 100 | | | 100 | |
| 72 | 8 | 90 | | 90 | 100 | | | 100 | | | 100 | |
| | 1 | 10 | | 80 | 80 | | | 100 | | | 100 | |
| 73 | 8 | 60 | | 100 | 100 | | | 100 | | | 100 | |
| | 1 | 20 | | 80 | 80 | | | 100 | | | 95 | |
| 74 | 8 | 10 | | 0 | 80 | | | 100 | | | 80 | |
| 75 | 8 | 95 | | 100 | 95 | | | 100 | | | 100 | |
| | 2 | 0 | 100 | 80 | 30 | 100 | 20 | 100 | 98 | 100 | 100 | 100 |
| 76 | 8 | 100 | | 100 | 100 | | | 100 | | | 100 | |
| | 1 | 10 | 100 | 90 | 60 | 100 | 70 | 100 | 90 | 100 | 100 | 100 |
| 77 | 8 | 0 | | 40 | 20 | | | 100 | | | 0 | |
| 88 | 8 | 100 | | 100 | 100 | | | 100 | | | 100 | |
| | 1 | 60 | | 100 | 100 | | | 100 | | | 100 | |
| 89 | 8 | 100 | | 100 | 100 | | | 100 | | | 100 | |
| | 1 | 95 | | 100 | 95 | | | 100 | | | 100 | |
| 90 | 8 | 100 | | 100 | 100 | | | 100 | | | 100 | |
| | 1 | 95 | | 98 | 98 | | | 100 | | | 100 | |
| 91 | 8 | 60 | | 100 | 100 | | | 100 | | | 100 | |
| | 1 | 20 | | 98 | 98 | | | 100 | | | 100 | |

Tableau (IX)

| Composé no | Dose kg/ha | Cultures en prélevée | | | | | | | | |
|---|---|---|---|---|---|---|---|---|---|---|
| | | BLE | MAI | ORG | RIZ | COL | COT | HAR | SOJ | TOU |
| 51 | 8 | | | | | | | 0 | | |
| | 2 | 0 | 5 | 0 | 0 | | 0 | 0 | 50 | 0 |
| 52 | 8 | | | | | | | 0 | | |
| | 1 | 20 | 0 | 15 | 0 | | 0 | 0 | 0 | 0 |
| 53 | 8 | | | | | | | 0 | | |
| | 1 | 0 | 0 | 0 | 15 | 10 | 0 | 0 | 0 | 0 |
| 54 | 8 | | | | | | | 30 | | |
| | 0,5 | 0 | 0 | 0 | 5 | | 0 | 0 | 0 | 0 |
| 55 | 0,5 | 0 | 0 | 0 | 10 | | 0 | 30 | 10 | 0 |
| 56 | 8 | | | | | | | 30 | | |
| 57 | 0,5 | 0 | 0 | 0 | 0 | | 0 | 0 | 0 | 0 |

Tableau (IX) (suite)

| Composé no | Dose kg/ha | Cultures en prélevée | | | | | | | | |
|---|---|---|---|---|---|---|---|---|---|---|
| | | BLE | MAI | ORG | RIZ | COL | COT | HAR | SOJ | TOU |
| 58 | 2 | 0 | 10 | 0 | 30 | | 0 | | 0 | 0 |
| 59 | 2 | 5 | 10 | 0 | 0 | | 0 | 0 | 5 | 0 |
| 60 | | | | | | | | | | |
| | 0,5 | 0 | 20 | 0 | 0 | | 0 | 30 | 0 | 0 |
| 61 | 8 | | | | | | | 70 | | |
| 62 | 0,5 | 0 | 0 | 0 | 10 | 20 | 0 | 0 | 10 | 0 |
| 63 | 8 | | | | | | | | | |
| 64 | 8 | | | | | | | | | |
| | 2 | | | | | | | | | |
| 65 | 8 | | | | | | | | | |
| | 2 | | | | | | | | | |
| 66 | 1 | 0 | 0 | 0 | 10 | 0 | 0 | 0 | 50 | 0 |
| 67 | 1 | 0 | 0 | 0 | 5 | | 0 | 0 | 0 | 0 |
| 68 | 1 | | | | | | | 30 | | |
| 69 | 2 | 0 | 0 | 0 | 10 | | 0 | 0 | 0 | 0 |
| 70 | 1 | 0 | 60 | 20 | 10 | | 0 | 0 | 0 | 0 |
| 71 | 8 | | | | | | | 30 | | |
| 72 | 1 | | | | | | | 30 | | |
| 73 | 1 | | | | | | | 30 | | |
| 74 | 8 | | | | | | | 0 | | |
| 75 | 2 | 0 | 20 | 10 | 0 | | 0 | 50 | 20 | 0 |
| 76 | 1 | 5 | 20 | 0 | 20 | | 0 | 0 | 0 | 0 |
| 77 | 8 | | | | | | | 0 | | |

Tableau (X)

| Composé no | Dose kg/ha | Adventices ou cultures (traitement en postlevée) | | | | | |
|---|---|---|---|---|---|---|---|
| | | FAV | PAN | RAY | CHE | MOU | HAR |
| 1 | 8 | 100 | 100 | 100 | 60 | 50 | 0 |
| 2 | 8 | 80 | 90 | 50 | 30 | 60 | |
| 4 | 8 | 30 | 90 | 30 | 20 | 40 | |
| 5 | 8 | 10 | 90 | 60 | 0 | 30 | |
| 6 | 8 | 20 | 95 | 80 | 0 | 30 | |
| 7 | 8 | 30 | 80 | 80 | 0 | 20 | 0 |
| 8 | 8 | 30 | 30 | 30 | 0 | 20 | |

Tableau (X) (suite)

| Composé no | Dose kg/ha | Adventices ou cultures (traitement en postlevée) | | | | | |
|---|---|---|---|---|---|---|---|
| | | FAV | PAN | RAY | CHE | MOU | HAR |
| 10 | 8 | 50 | 100 | 80 | 0 | 0 | |
| 12 | 8 | 30 | 80 | 0 | 30 | 20 | 0 |
| 13 | 8 | 20 | 20 | 60 | 0 | 10 | 0 |
| 14 | 8 | 100 | 100 | 100 | 30 | 70 | |
| 15 | 8 | 70 | 100 | 90 | 20 | 90 | |
| 16 | 8 | 0 | 40 | 0 | 20 | 0 | 0 |
| 17 | 8 | 0 | 40 | 20 | 20 | 0 | 0 |
| 18 | 8 | 20 | 60 | 30 | 20 | 0 | 0 |
| 21 | 8 | 100 | 100 | 100 | 30 | 50 | |
| 22 | 8 | 98 | 100 | 100 | 80 | 100 | |
| 23 | 8 | 30 | 20 | 20 | 30 | 20 | 0 |
| 24 | 8 | 95 | 100 | 80 | 30 | 50 | 0 |
| 25 | 8 | 20 | 60 | 30 | 20 | 20 | |
| 26 | 8 | 30 | 100 | 30 | 20 | 30 | 0 |
| 27 | 8 | 0 | 95 | 70 | 20 | 20 | 0 |
| 28 | 8 | 20 | 100 | 20 | 30 | 80 | 0 |
| 29 | 8 | 0 | 90 | 20 | 20 | 30 | |
| 30 | 8 | 0 | 0 | 0 | 20 | 0 | 0 |
| 31 | 8 | 100 | 95 | 95 | 30 | 30 | |

Tableau (XI)

| Composé no | Dose kg/ha | Adventices ou cultures, en postlevée | | | | |
|---|---|---|---|---|---|---|
| | | FAV | PAN | RAY | MOU | CHE |
| 51 | 8 | 20 | 30 | 40 | 0 | 0 |
| 52 | 8 | 40 | 95 | 90 | 0 | 0 |
| 53 | 8 | 70 | 100 | 90 | 30 | 20 |
| 54 | 8 | 60 | 100 | 80 | 90 | 30 |
| 55 | 8 | 30 | 100 | 20 | 20 | 20 |
| 56 | 8 | 0 | 70 | 20 | 0 | 0 |
| 57 | 8 | 100 | 100 | 98 | 30 | 30 |
| 58 | 8 | 50 | 100 | 90 | 80 | 30 |
| 59 | 8 | 70 | 100 | 40 | 30 | 30 |
| 60 | 8 | 98 | 100 | 100 | 90 | 80 |

Tableau (XI) (suite)

| Composé no | Dose kg/ha | Adventices ou cultures, en postlevée | | | | |
|---|---|---|---|---|---|---|
| | | FAV | PAN | RAY | MOU | CHE |
| 61 | 8 | 20 | 100 | 30 | 90 | 20 |
| 62 | 8 | 20 | 98 | 50 | 70 | 30 |
| 63 | 8 | 10 | 100 | 30 | 100 | 80 |
| 64 | 8 | 0 | 98 | 95 | 30 | 20 |
| 65 | 8 | 95 | 100 | 100 | 70 | 100 |
| | 2 | 50 | 100 | 95 | 20 | 70 |
| 66 | 8 | 0 | 50 | 20 | 0 | 40 |
| 67 | 8 | 0 | 50 | 30 | 0 | 20 |
| 68 | 8 | 30 | 80 | 20 | 40 | 20 |
| 69 | 8 | 0 | 100 | 50 | 30 | 30 |
| 70 | 8 | 98 | 100 | 100 | 60 | 80 |
| 72 | 8 | 10 | 95 | 80 | 30 | 5 |
| 73 | 8 | 0 | 95 | 80 | 60 | 0 |
| 88 | 1 | 30 | 98 | 80 | 20 | 20 |
| 89 | 1 | 30 | 95 | 20 | 20 | 20 |
| 90 | 1 | 30 | 60 | 70 | 0 | 50 |
| 91 | 1 | 30 | 80 | 30 | 30 | 30 |

Tableau (XII)

| Composé no | Dose kg/ha | Adventices ou cultures (traitement en postlevée) | | | | | | | | | | | | | | |
|---|---|---|---|---|---|---|---|---|---|---|---|---|---|---|---|---|
| | | BLE | ORG | RIZ | COL | COT | HAR | SOJ | TOU | DIG | PAN | RAY | SET | VUL | MOR | STE |
| 10 | 8 | 5 | 15 | 10 | 10 | 0 | 0 | 30 | 0 | 90 | 80 | 60 | 100 | 90 | 100 | 20 |
| 14 | 2 | 0 | 20 | 0 | 0 | 20 | 0 | 5 | 5 | 60 | 0 | 0 | 20 | 60 | 10 | 100 |
| 15 | 4 | 0 | | | 20 | 0 | 0 | 5 | 0 | 60 | 80 | 0 | 80 | 10 | 90 | 0 |
| 21 | 1 | 10 | 10 | 10 | 0 | 20 | 0 | 0 | 5 | 20 | 80 | 20 | 60 | 95 | 80 | 100 |
| 22 | 1 | 0 | 5 | 5 | 0 | 0 | 0 | 10 | 20 | 95 | 95 | 20 | 100 | 70 | 80 | 0 |
| 53 | 4 | 0 | 0 | 0 | 20 | 0 | 0 | 0 | 10 | 80 | 90 | 10 | 85 | 60 | 95 | 10 |
| 54 | 4 | 0 | 5 | 5 | 20 | 0 | 0 | 20 | 0 | 80 | 60 | 0 | 80 | 10 | 30 | 10 |
| 60 | 1 | 0 | 20 | 5 | 0 | 0 | 0 | 0 | 5 | 100 | 90 | 10 | 95 | 0 | 90 | 0 |
| 61 | 4 | 0 | 0 | 0 | 0 | 0 | 0 | 0 | 0 | 20 | 30 | 10 | 90 | 10 | 5 | 0 |
| 63 | 4 | 0 | 0 | 0 | 0 | 20 | 0 | 5 | 0 | 0 | 60 | 0 | 20 | 0 | 90 | 60 |
| 65 | 2 | 0 | 0 | 0 | 10 | 0 | 0 | 0 | 5 | 20 | 80 | 10 | 100 | 10 | 100 | 40 |
| 70 | 1 | 0 | 20 | 10 | 0 | 0 | 0 | 0 | 0 | 50 | 80 | 5 | 10 | 80 | 10 | 80 |

**Revendications pour les Etats contractants BE CH DE FR GB IT LI LU NL SE**

1. Composés, utilisables notamment comme herbicides, caractérisés en ce qu'ils ont pour formule

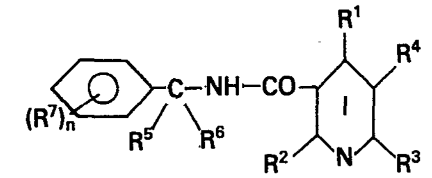

(I)

dans laquelle:

$R^1$, $R^2$ et $R^3$, identiques ou différents, représentent l'atome d'hydrogène ou un radical alkyle ayant au plus 6 atomes de carbone, ou un radical alkoxy ayant au plus 6 atomes de carbone, ou un radical alkoxyalkyle ayant de 2 à 6 atomes de carbone,

$R^4$ représente le radical carboxy, éventuellement sous forme sel ou ester ou le radical cyano, ou un radical cyanoalkyle ayant au plus 6 atomes de carbone, ou un radical alkoxyalkyle ayant de 2 à 8 atomes de carbone,

$R^5$ et $R^6$ représentent l'atome d'hydrogène ou un radical alkyle ayant au plus 6 atomes de carbone, ou constituent ensemble un radical unique divalent alkylène ayant de 2 à 5 atomes de carbone, l'un de ces deux radicaux $R^5$ et $R^6$ pouvant en outre représenter un radical cyano,

$R^7$ représente un atome d'halogène ou un radical alkyle ayant au plus 6 atomes de carbone, ou un radical alkoxy ayant au plus 6 atomes de carbone ou un radical alcényle ayant au plus 6 atomes de carbone, ou un radical alcényloxy ayant au plus 6 atomes de carbone (ces divers radicaux alkyle, alkoxy, alcényle, alcényloxy étant éventuellement halogénés) ou un radical nitro ou cyano, ou un radical amino, ou deux $R^7$ forment ensemble un radical alkylènedioxy comportant de 1 à 4 atomes de carbone,

n est un nombre entier, positif ou nul, au plus égal à 5.

L'hétérocycle

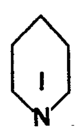

représente un hétérocycle azoté insaturé à 6 côtés et contenant 2 ou 3 insaturations.

2. Composés selon la revendication 1 caractérisés en ce que

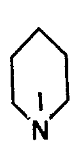

représente le noyau dihydropyridinique

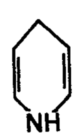

3. Composés selon la revendication 1 caractérisés en ce que

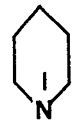

représente le noyau pyridinique

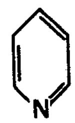

4. Composés selon l'une des revendication 1 à 3 caractérisés en ce que $R^4$ représente un radical $-COOR^8$, $R^8$ étant un radical alkyle comportant au plus six atomes de carbone ou $R^4$ représente un radical alkoxyalkyle ou un radical cyanoalkyle.

5. Composés selon l'une des revendications 1 à 4 caractérisés en ce que

$R^1$ est l'atome d'hydrogène,

$R^2$ et $R^3$ sont le radical méthyle ou méthoxy ou méthoxyméthyle,

$R^4$ est $COOR^8$, $R^8$ ayant de 1 à 4 atomes de carbone ou $R_4$ est un radical cyanométhyle ou alkoxyméthyle, ayant au plus 6 atomes de carbone,

$R^5$ et $R^6$ sont l'atome d'hydrogène ou, pour l'un d'entre eux, le radical méthyle ou forment ensemble une chaîne éthylène,

$R^7$ est un atome de chlore ou de fluor, ou un radical alkyle ayant de 1 à 4 atomes de carbone et éventuellement halogéné, ou un radical alkoxy ayant de 1 à 4 atomes de carbone,

n est égal à 0, 1 ou 2.

6. Composés selon l'une des revendications 1 à 5 caractérisés en ce qu'ils sont sous forme d'isomère optique de même configuration que la (1)-α-méthyl benzylamine.

7. Procédé de préparation de composés selon l'une des revendications 2 et 4 à 6 caractérisé en ce qu'on fait réagir ensemble un aldéhyde de formule $R^1-CHO$ avec un dérivé aminoéthylénique de formule (IV) et avec un amide cétonique de formule (V):

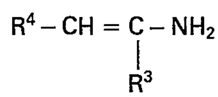

(IV)

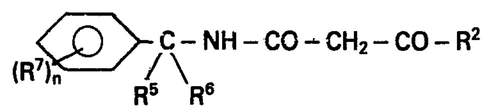

(V)

dans lesquelles $R^1$, $R^2$, $R^3$, $R^4$, $R^5$, $R^6$, $R^7$ et n ont les significations données dans les revendications 2 et 4 à 6.

8. Procédé selon la revendication 7 caractérisé en ce que la réaction est effectuée entre 15 et 100°C en présence d'un solvant inerte.

9. Procédé selon l'une des revendications 7 ou 8 caractérisé en ce que la concentration en réactifs est supérieure à 5% en poids et que leur proportions relatives s'écartent de moins de 10% (en mole) de la stoechiométrie.

10. Procédé de préparation de composés selon l'une des revendications 3 à 6 caractérisé en ce qu'on déshydrogène un composé selon la revendication 2.

11. Procédé selon la revendication 10 caractérisé en ce que la déshydrogénation se fait au moyen d'un oxydant tel que les acides nitrique ou nitreux ou chromique, l'iode, le soufre.

12. Procédé selon la revendication 10 caractérisé en ce que la déshydrogénation s'effectue par simple chauffage sous atmosphère contenant de l'oxygène, éventuellement en présence d'un catalyseur de déshydrogénation.

13. Procédé de préparation d'un composé selon la revendication 1, pour lequel

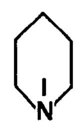

représente le noyau pyridinique et $R_4$ un radical cyanoalkyle caractérisé en ce qu'il consiste à faire réagir le cyanure de sodium sur la chloralkylepyridine de formule:

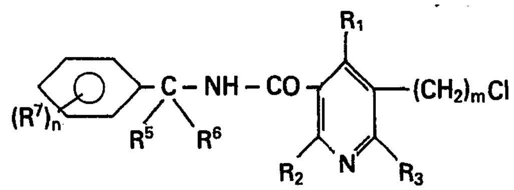

dans laquelle $R_1$, $R_2$, $R_3$, $R_5$, $R_6$, $R_7$ et n ont la même signification que dans la revendication 1 et m représente un nombre entier de 1 à 4.

14. Procédé de préparation d'un composé selon la revendication 1, pour lequel

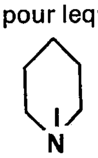

représente le noyau pyridinique et $R_4$ un radical alkoxyalkyle caractérisé en ce qu'il consiste à faire réagir un alcanolate alcalin sur la chloroalkyl-pyridine de formule:

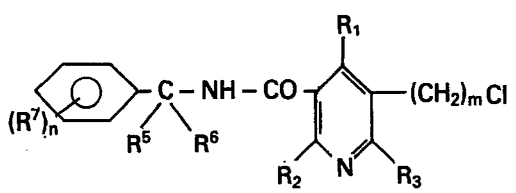

dans laquelle $R_1$, $R_2$, $R_3$, $R_5$, $R_6$, $R_7$ et n ont la même signification que dans la revendication 1 et m représente un nombre entier de 1 à 4.

15. Composition herbicide caractérisée en ce qu'elle contient, comme matière active, au moins un composé selon l'une des revendications 1 à 6.

16. Composition selon la revendication 15 caractérisé en ce qu'elle contient 0,05 à 95% en poids de matière active.

17. Compositon selon l'une des revendications 15 à 16 caractérisé en ce qu'elle contient en outre 1 à 94,95% d'un support solide ou liquide et, éventuellement, 0,1 à 20% d'agent(s) tensio-actif(s).

18. Procédé de désherbage sélectif des cultures, notamment de coton, tournesol, blé ou orge caractérisé en ce qu'on applique sur les plantes et/ou sur le sol de la zone à désherber une quantité efficace d'au moins un composé selon l'une des revendications 1 à 6.

19. Procédé selon la revendication 18 caractérisé en ce qu'on applique entre 0,1 et 10 kg/ha de la matière active.

20. Procédé selon l'une des revendications 18 ou 19 caractérisé en ce que l'application s'effectue en prélevée des cultures ou en présemis des cultures avec incorporation.

**Revendications pour l'Etat contractant AT**

1. Composition herbicide caractérisée en ce qu'elle contient comme matière active au moins un composè selon la formule (I) ci-après:

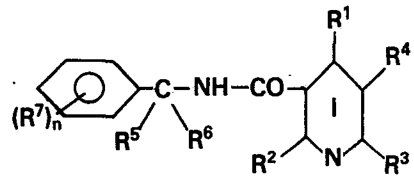

(I)

dans laquelle:

$R^1$, $R^2$ et $R^3$, identiques ou différents, représentent l'atome d'hydrogène ou un radical alkyle ayant au plus 6 atomes de carbone, ou un radical alkoxy ayant au plus 6 atomes de carbone, ou un radical alkoxyalkyle ayant de 2 à 8 atomes de carbone,

$R^4$ représente le radical carboxy, éventuellement sous forme sel ou ester ou le radical cyano, ou un radical cyanoalkyle ayant au plus 6 atomes de carbone, ou un radical alkoxyalkyle ayant de 2 à 8 atomes de carbone,

$R^5$ et $R^6$ représentent l'atome d'hydrogène ou un radical alkyle ayant au plus 6 atomes de carbone, ou constituent ensemble un radical unique divalent alkylène ayant de 2 à 5 atomes de carbone, l'un de ces radicaux $R^5$ et $R^6$ pouvant en outre représenter un radical cyano,

$R^7$ représente un atome d'halogène ou un radical alkyle ayant au plus 6 atomes de carbone, ou un radical alkoxy ayant au plus 6 atomes de carbone ou un radical alcényle ayant au plus 6 atomes de carbone, ou un radical alcényloxy ayant au plus 6 atomes de carbone (ces divers radicaux alkyle, alkoxy, alcényle, alcényloxy étant éventuellement halogénés) ou un radical nitro ou cyano, ou un radical amino éventuellement substitué, ou deux $R^7$ forment ensemble un radical alkylène-dioxy comportant de 1 à 4 atomes de carbone,

n est un nombre entier, positif ou nul, au plus égal à 5.

L'hétérocycle

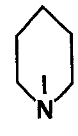

représente un hétérocycle azoté insaturé à 6 côtés pouvant contenant 2 ou 3 insaturations.

2.Composition selon la revendication 1 caractérisée en ce que, dans la formule (I), l'hétérocycle

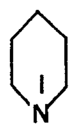

représente le noyau dihydropyridinique

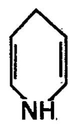

3. Composition selon la revendication 1 caractérisée en ce que, dans la formule (I), l'hétérocycle

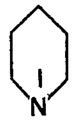

représente le noyau pyridinique

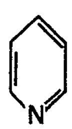

4. Composition selon l'une des revendication 1 à 3 caractérisée en ce que, dans la formfule (I), $R^4$ représente un radical $-COOR^8$, $R^8$ étant un radical alkyle ayant au plus 6 atomes de carbone, ou $R^4$ représente un radical cyanoalkyle ou alkoxyalkyle.

5. Composition selon l'une des revendications 1 à 4 caractérisée en ce que, dans la formule (I)

$R^1$ est l'atome d'hydrogène,

$R^2$ et $R^3$ sont le radical méthyle ou méthoxy ou méthoxyméthyle,

$R^4$ est $COOR^8$, $R^8$ ayant de 1 à 4 atomes de carbone ou $R_4$ est un radical cyanométhyle ou alkoxyméthyle, ayant au plus 6 atomes de carbone,

$R^5$ et $R^6$ sont l'atome d'hydrogène ou, pour l'un d'entre eux, le radical méthyle ou forment ensemble une chaîne éthylène,

$R^7$ est un atome de chlore ou de fluor, ou un radical alkyle ayant de 1 à 4 atomes de carbone et éventuellement halogéné, ou un radical alkoxy ayant de 1 à 4 atomes de carbone,

n est égal à 0, 1 ou 2.

6. Composition selon l'une des revendications 1 à 5 caractérisée en ce que le composé de formule (I) est sous forme d'isomère optique de même configuration que la (1)-α-méthyl benzylamine.

7. Composition selon l'une des revendications 1 à 6 caractérisée en ce qu'elle contient 0,05 à 95% en poids de matière active.

8. Compositon selon la revendication 7 caractérisée en ce qu'elle contient en outre 1 à 94,95% d'un support solide ou liquide et, éventuellement, 0,1 à 20% d'agent(s) tensio-actif(s).

9. Procédé de préparation d'un composé selon la formule (I) figurant dans la revendications 1, dans laquelle $R^1$, $R^2$, $R^3$, $R^4$, $R^5$, $R^6$, $R^7$ et n ont la même signification que dans la revendication 1 et l'hétérocycle

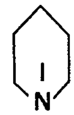

représente le noyau dihydropyridinique

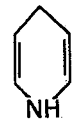

caractérisée en ce qu'on fait réagir ensemble un aldéhyde de formule $R^1$–CHO avec un dérivé aminoéthylénique de formule (IV) et avec un amide cétonique de formule (V):

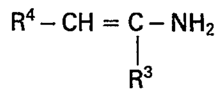

(IV)

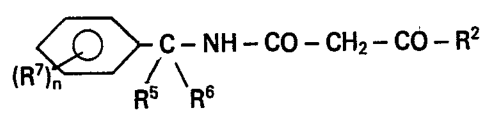

(V)

dans lesquelles $R^1$, $R^2$, $R^3$, $R^4$, $R^5$, $R^6$, $R^7$ et n ont les significations données précédemment.

10. Procédé selon la revendication 9 caractérisé en ce que la réaction est effectuée entre 15 et 100°C en présence d'un solvant inerte.

11. Procédé selon l'une des revendications 9 ou 10 caractérisé en ce que la concentration en réactifs est supérieure à 5% en poids et que leur proportions relatives s'écartent de moins de 10% (en mole) de la stoechiométrie.

12. Procédé de préparation d'un composé selon la formule (I) figurant dans la revendication 1, dans laquelle $R^1$, $R^2$, $R^3$, $R^4$, $R^5$, $R^6$, $R^7$ et n ont la même signification que dans la revendication 1 et l'hétérocycle:

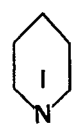

représente le noyau pyridinique:

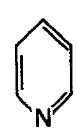

caractérisé en ce qu'on déshydrogène le produit obtenu selon la revendication 9.

13. Procédé selon la revendication 12 caractérisé en ce que la déshydrogénation se fait au moyen d'un oxydant tel que les acides nitrique ou nitreux ou chromique, l'iode, le soufre.

14. Procédé selon la revendication 13 caractérisé en ce que la déshydrogénation s'effectue par simple chauffage sous atmosphère contenant de l'oxygène, éventuellement en présence d'un catalyseur de déshydrogénation.

15. Procédé de préparation d'un composé selon la formule (I) figurant dans la revendication 1,

dans laquelle $R^1$, $R^2$, $R^3$, $R^5$, $R^6$, $R^7$ et n ont la même signification que dans la revendication 1, l'hétérocycle

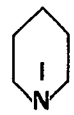

représente le noyau pyridinique

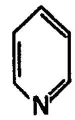

et $R^4$ représente un radical cyanoalkyle, caractérisé en ce qu'il consiste à faire réagir le cyanure de sodium sur la chloralkyle-pyridine de formule:

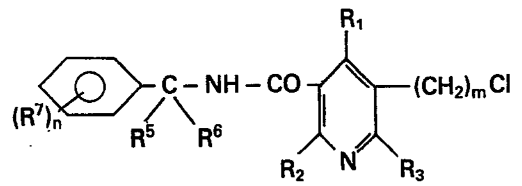

dans laquelle $R^1$, $R^2$, $R^3$, $R^5$, $R^6$, $R^7$ et n ont la même signification que dans la revendication 1 et m représente un nombre entier de 1 à 4.

16. Procédé de préparation d'un composé selon la formule (I) figurant dans la revendication 1, dans laquelle $R^1$, $R^2$, $R^3$, $R^5$, $R^6$, $R^7$ et n ont la même signification que dans la revendication 1, l'hétérocycle

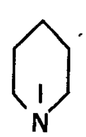

représente le noyau pyridinique

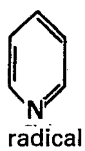

et $R_4$ représente un radical alkoxyalkyle, caractérisé en ce qu'il consiste à faire réagir un alcanolate alcalin sur la chloralkyle-pyridine de formule:

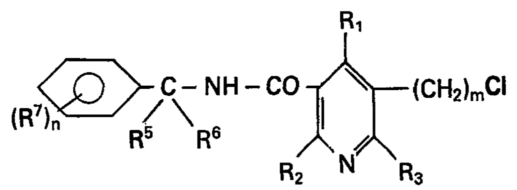

dans laquelle $R_1$, $R_2$, $R_3$, $R_5$, $R_6$, $R_7$ et n ont la même signification que dans la formule (I) de la revendication 1 et m représente un nombre entier de 1 à 4.

17. Procédé de désherbage sélectif des cultures, notamment de coton, tournesol, blé ou orge caractérisé en ce qu'on applique sur les plantes et/ou sur le sol de la zone à désherber une quantité efficace d'une composition selon l'une des revendications 1 à 8.

18. Procédé selon la revendication 17 caractérisé en ce qu'on applique entre 0,1 et 10 kg/ha de la matière active.

19. Procédé selon l'une des revendications 17 ou 18 caractérisé en ce que l'application s'effectue en prélevée des cultures ou en présemis des cultures avec incorporation.

**Patentansprüche für die Vertragsstaaten BE CH DE FR GB IT LI LU NL SE**

1. Vor allem als Herbizide brauchbare Verbindungen, dadurch gekennzeichnet, dass sie der Formel

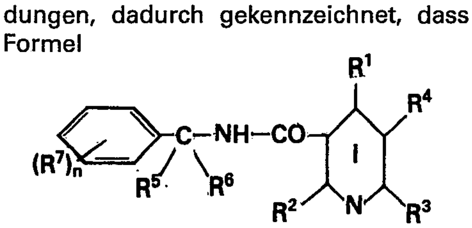

(I)

entsprechen, in der:

$R^1$, $R^2$ und $R^3$ gleich oder verschieden sind und ein Wasserstoffatom oder eine Alkylgruppe mit maximal 6 Kohlenstoffatomen, oder eine Alkoxygruppe mit maximal 6 Kohlenstoffatomen oder eine Alkoxyalkylgruppe mit 2 bis 8 Kohlenstoffatomen bedeuten,

$R^4$ für die Carboxylgruppe, gegebenenfalls in Form ihres Salzes oder Esters, oder für eine Cyanogruppe oder eine Cyanoalkylgruppe mit maximal 6 Kohlenstoffatomen oder für eine Alkoxyalkylgruppe mit 2 bis 8 Kohlenstoffatomen steht,

$R^5$ und $R^6$ (jeweils) ein Wasserstoffatom oder eine Alkylgruppe mit maximal 6 Kohlenstoffatomen bedeuten oder zusammen eine einzige zweiwertige Alkylengruppe mit 2 bis 5 Kohlenstoffatomen bilden und einer der beiden Substituenten $R^5$ und $R^6$ ausserdem eine Cyanogruppe sein kann,

$R^7$ ein Halogenatom oder eine Alkylgruppe mit maximal 6 Kohlenstoffatomen oder eine Alkoxygruppe mit maximal 6 Kohlenstoffatomen oder eine Alkenylgruppe mit maximal 6 Kohlenstoffatomen oder eine Alkenyloxygruppe mit maximal 6 Kohlenstoffatomen ist, (wobei diese verschiedenen Alkyl-, Alkoxy-, Alkenyl- und Alkenyloxygruppen gegebenenfalls halogeniert sind) oder eine Nitro- oder Cyanogruppe oder eine Aminogruppe bedeutet, oder zwei $R^7$ zusammen eine Alkylendioxygruppe mit 1 bis 4 Kohlenstoffatomen bilden können,

n eine ganze positive Zahl von maximal 5 oder 0 ist,

der Heterocyclus

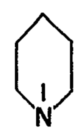

einen stickstoffhaltigen, 6-gliedrigen Heterocyclus bedeutet, der 2 oder 3 Doppelbindungen enthält.

2. Verbindungen nach Anspruch 1, dadurch gekennzeichnet, dass

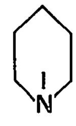

für den Dihydropyridinring

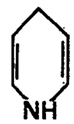

steht.

3. Verbindungen nach Anspruch 1, dadurch gekennzeichnet, dass

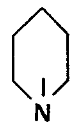

für den Pyridinring

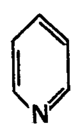

steht.

4. Verbindungen nach einem der Ansprüche 1 bis 3, dadurch gekennzeichnet, dass $R^4$ eine Gruppe $-COOR^8$ bedeutet, in der $R^8$ eine Alkylgruppe mit maximal 6 Kohlenstoffatomen ist oder $R^4$ eine Alkoxyalkylgruppe oder eine Cyanoalkylgruppe ist.

5. Verbindungen nach einem der Ansprüche 1 bis 4, dadurch gekennzeichnet, dass

- $R^1$ ein Wasserstoffatom ist,
- $R^2$ und $R^3$ für eine Methyl-, Methoxy- oder Methoxymethylgruppe stehen,
- $R^4$ die Gruppe $-COOR^8$ ist, wobei $R^8$ 1 bis 4 Kohlenstoffatome enthält, oder $R^4$ eine Cyanomethyl- oder Alkoxymethylgruppe mit maximal 6 Kohlenstoffatomen (im Alkylrest) ist,
- $R^5$ und $R^6$ (jeweils) ein Wasserstoffatom sind oder einer dieser beiden (Substituenten) für die Methylgruppe steht, oder gemeinsam eine Ethylengruppe bilden,
- $R^7$ ein Chlor- oder Fluoratom oder eine Alkylgruppe mit 1 bis 4 Kohlenstoffatomen, gegebenenfalls halogeniert, oder eine Alkoxygruppe mit 1 bis 4 Kohlenstoffatomen bedeuten,
- n 0, 1 oder 2 ist.

6. Verbindungen nach einem der Ansprüche 1 bis 5, dadurch gekennzeichnet, dass sie als optisches Isomer der gleichen Konfiguration wie (1)-α-Methylbenzylamin vorliegen.

7. Verfahren zur Herstellung von Verbindungen nach einem der Ansprüche 2 bis 4 und 6, dadurch gekennzeichnet, dass man einen Aldehyd der Formel $R^1$–CHO mit einer Aminoethylenverbindung der allgemeinen Formel

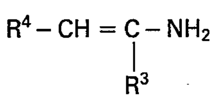 (IV)

und mit einem Keto-amid der allgemeinen Formel

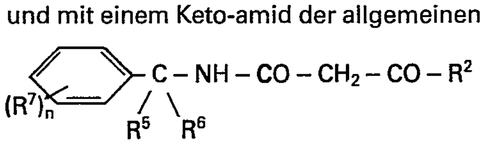 (V)

in denen $R^1$, $R^2$, $R^3$, $R^4$, $R^5$, $R^6$, $R^7$ und n die in den Ansprüchen 2 und 4 bis 6 angegebenen Bedeutungen haben, umsetzt.

8. Verfahren nach Anspruch 7, dadurch gekennzeichnet, dass man die Umsetzung zwischen 15 und 100°C in Gegenwart eines inerten Lösungsmittels vornimmt.

9. Verfahren nach einem der Ansprüche 7 oder 8, dadurch gekennzeichnet, dass die Konzentration der Reaktionspartner mehr als 5 Gew.-% beträgt und dass ihre relativen Mengenanteile um weniger als 10 mol-% vom stöchiometrischen Verhältnis abweichen.

10. Verfahren zur Herstellung von Verbindungen nach einem der Ansprüche 3 bis 6, dadurch gekennzeichnet, dass man eine Verbindung nach Anspruch 2 dehydriert.

11. Verfahren nach Anspruch 10, dadurch gekennzeichnet, dass man die Dehydrierung mit einem Oxidationsmittel wie Salpetersäure oder Salpetrige Säure oder Chromsäure, Iod oder Schwefel vornimmt.

12. Verfahren nach Anspruch 10, dadurch gekennzeichnet, dass man die Dehydrierung durch einfaches Erhitzen in sauerstoffhaltiger Atmosphäre, gegebenenfalls in Gegenwart eines Dehydrierungskatalysators durchführt.

13. Verfahren zur Herstellung einer Verbindung nach Anspruch 1, bei der

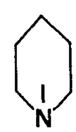

den Pyridinring und $R^4$ eine Cyanoalkylgruppe bedeutet, dadurch gekennzeichnet, dass man Natriumcyanid mit einem Chloralkylpyridin der Formel

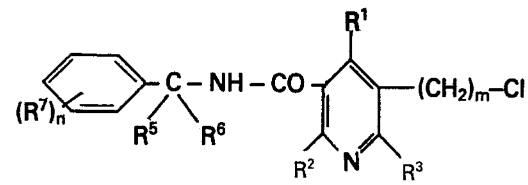

in der $R^1$, $R^2$, $R^3$, $R^5$, $R^6$, $R^7$ und n die gleiche Bedeutung haben wie in Anspruch 1 und n eine ganze Zahl von 1 bis 4 ist, umsetzt.

14. Verfahren zur Herstellung einer Verbindung nach Anspruch 1, bei der

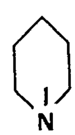

den Pyridinring und $R^4$ eine Alkoxyalkylgruppe bedeutet, dadurch gekennzeichnet, dass man ein Alkalialkanolat mit einem Chloralkylpyridin der Formel

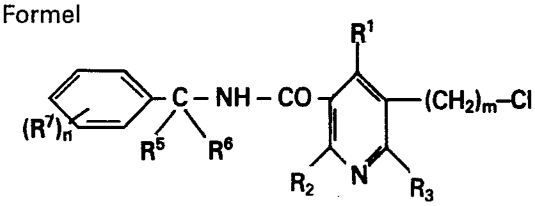

in der $R^1$, $R^2$, $R^3$, $R^5$, $R^6$, $R^7$ und n die gleiche Bedeutung wie in Anspruch 1 haben und n eine ganze Zahl von 1 bis 4 ist, umsetzt.

15. Herbizides Mittel, dadurch gekennzeichnet, dass es als Wirkstoff mindestens eine Verbindung nach einem der Ansprüche 1 bis 6 enthält.

16. Mittel nach Anspruch 15, dadurch gekennzeichnet, dass es 0,05 bis 95 Gew.-% Wirkstoff enthält.

17. Mittel nach Anspruch 15 oder 16, dadurch gekennzeichnet, dass es zusätzlich 1 bis 94,95% eines festen oder flüssigen Trägers und, gegebenenfalls 0,1 bis 20% grenzflächenaktive(s) Mittel enthält.

18. Verfahren zur selektiven Unkrautvertilgung in Anpflanzungen, vor allem von Baumwolle, Sonnenblumen, Weizen oder Gerste, dadurch gekennzeichnet, dass man auf die Pflanzen und/oder auf den Boden des Bereiches, in dem das Unkraut vertilgt werden soll, eine wirksame Menge mindestens einer Verbindung nach einem der Ansprüche 1 bis 6 aufbringt.

19. Verfahren nach Anspruch 18, dadurch gekennzeichnet, dass man 0,1 bis 10 kg/ha Wirkstoff aufbringt.

20. Verfahren nach Anspruch 18 oder 19, dadurch gekennzeichnet, dass die Behandlung vor dem Auflaufen der Nutzpflanzen oder vor der Aussaat der Nutzpflanzen mit Einarbeiten (in den Boden) vorgenommen wird.

**Patentansprüche für den Vertragsstaat AT**

1. Herbizides Mittel, dadurch gekennzeichnet, dass es als Wirkstoff mindestens eine Verbindung der folgenden Formel I

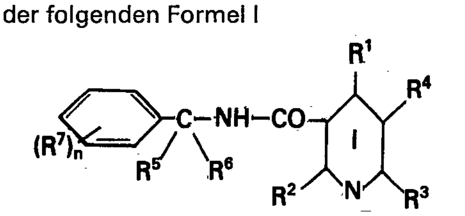

(I)

enthält, in der:

- $R^1$, $R^2$ und $R^3$ gleich oder verschieden sind und ein Wasserstoffatom oder eine Alkylgruppe mit maximal 6 Kohlenstoffatomen, oder eine Alkoxygruppe mit maximal 6 Kohlenstoffatomen oder eine Alkoxyalkylgruppe mit 2 bis 8 Kohlenstoffatomen bedeuten,
- $R^4$ für die Carboxylgruppe, gegebenenfalls in Form ihres Salzes oder Esters, oder für eine Cyanogruppe oder eine Cyanoalkylgruppe mit maximal 6 Kohlenstoffatomen oder für eine Alkoxyalkylgruppe mit 2 bis 8 Kohlenstoffatomen steht,
- $R^5$ und $R^6$ (jeweils) ein Wasserstoffatom oder eine Alkylgruppe mit maximal 6 Kohlenstoffatomen bedeuten oder zusammen eine einzige zweiwertige Alkylengruppe mit 2 bis 5 Kohlenstoffatomen bilden und einer der beiden Substituenten $R^5$ und $R^6$ ausserdem eine Cyanogruppe sein kann,
- $R^7$ ein Halogenatom oder eine Alkylgruppe mit maximal 6 Kohlenstoffatomen oder eine Alkoxygruppe mit maximal 6 Kohlenstoffatomen oder eine Alkenylgruppe mit maximal 6 Kohlenstoffatomen oder eine Alkenyloxygruppe mit maximal 6 Kohlenstoffatomen ist, (wobei diese verschiedenen Alkyl-, Alkoxy-, Alkenyl- und Alkenyloxygruppen gegebenenfalls halogeniert sind) oder eine Nitro- oder Cyanogruppe oder eine gegebenenfalls substituierte Aminogruppe bedeutet, oder zwei $R^7$ zusammen eine Alkylendioxygruppe mit 1 bis 4 Kohlenstoffatomen bilden können,
- n eine ganze positive Zahl von maximal 5 oder 0 ist,

der Heterocyclus

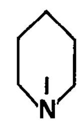

einen stickstoffhaltigen, 6-gliedrigen Heterocyclus bedeutet, der 2 oder 3 Doppelbindungen enthält.

2. Mittel nach Anspruch 1, dadurch gekennzeichnet, dass in der Formel I der Heterocyclus

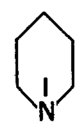

für den Dihydropyridinring

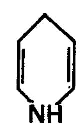

steht.

3. Mittel nach Anspruch 1, dadurch gekennzeichnet, dass in der Formel I der Heterocyclus

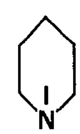

für den Pyridinring

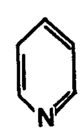

steht.

4. Mittel nach einem der Ansprüche 1 bis 3, dadurch gekennzeichnet, dass in der Formel I $R^4$ eine Gruppe $-COOR^8$ bedeutet, in der $R^8$ eine Alkylgruppe mit maximal 6 Kohlenstoffatomen ist, oder $R^4$ eine Alkoxyalkylgruppe oder eine Cyanoalkylgruppe ist.

5. Mittel nach einem der Ansprüche 1 bis 4, dadurch gekennzeichnet, dass in der Formel I

- $R^1$ ein Wasserstoffatom ist,
- $R^2$ und $R^3$ für eine Methyl-, Methoxy- oder Methoxymethylgruppe stehen,
- $R^4$ die Gruppe $-COOR^8$ ist, wobei $R^8$ 1 bis 4 Kohlenstoffatome enthält, oder $R^4$ eine Cyanomethyl- oder Alkoxymethylgruppe mit maximal 6 Kohlenstoffatomen (im Alkylrest) ist,

$R^5$ und $R^6$ (jeweils) ein Wasserstoffatom sind oder einer dieser beiden (Substituenten) für die Methylgruppe steht, oder gemeinsam eine Ethylengruppe bilden,

$R^7$ ein Chlor- oder Fluoratom oder eine Alkylgruppe mit 1 bis 4 Kohlenstoffatomen, gegebenenfalls halogeniert, oder eine Alkoxygruppe mit 1 bis 4 Kohlenstoffatomen bedeuten,

n 0, 1 oder 2 ist.

6. Mittel nach einem der Ansprüche 1 bis 5, dadurch gekennzeichnet, dass die Verbindung der Formel I als optisches Isomer der gleichen Konfiguration wie (1)-α-Methylbenzylamin vorliegt.

7. Mittel nach einem der Ansprüche 1 bis 6, dadurch gekennzeichnet, dass es 0,05 bis 95 Gew.-% Wirkstoff enthält.

8. Mittel nach Anspruch 7, dadurch gekennzeichnet, dass es zusätzlich 1 bis 94,95% eines festen oder flüssigen Trägers und, gegebenenfalls 0,1 bis 20% grenzflächenaktive(s) Mittel enthält.

9. Verfahren zur Herstellung einer Verbindung der Formel I in Anspruch 1, bei der $R^1$, $R^2$, $R^3$, $R^4$, $R^5$, $R^6$, $R^7$ und n die gleiche Bedeutung wie in Anspruch 1 haben und der Heterocyclus

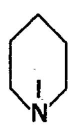

den Dihydropyridinring

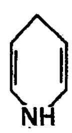

bedeutet, dadurch gekennzeichnet, dass man einen Aldehyd der Formel $R^1$–CHO mit einer Aminoethylenverbindung der Formel IV

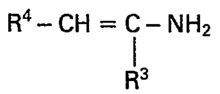

(IV)

und mit einem Keto-amid der Formel V

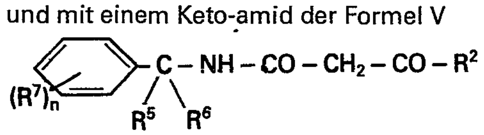

(V)

in denen $R^1$, $R^2$, $R^3$, $R^4$, $R^5$, $R^6$, $R^7$ und n die oben angebenenen Bedeutungen haben, umsetzt.

10. Verfahren nach Anspruch 9, dadurch gekennzeichnet, dass man die Umsetzung zwischen 15 und 100°C in Gegenwart eines inerten Lösungsmittels vornimmt.

11. Verfahren nach einem der Ansprüche 9 und 10, dadurch gekennzeichnet, dass die Konzentration der Reaktionspartner mehr als 5 Gew.-% beträgt und dass ihre relativen Mengenanteile um weniger als 10 mol-% vom stöchiometrischen Verhältnis abweichen.

12. Verfahren zur Herstellung einer Verbindung der Formel I in Anspruch 1, bei der $R^1$, $R^2$, $R^3$, $R^4$, $R^5$, $R^6$, $R^7$ und n die gleiche Bedeutung wie in Anspruch 1 haben und der Heterocyclus

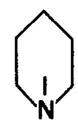

den Pyridinring

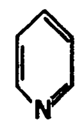

bedeutet, dadurch gekennzeichnet, dass man die nach Anspruch 9 erhaltene Verbindung dehydriert.

13. Verfahren nach Anspruch 12, dadurch gekennzeichnet, dass man die Dehydrierung mit einem Oxidationsmittel wie Salpetersäure oder Salpetrige Säure oder Chromsäure, Iod oder Schwefel vornimmt.

14. Verfahren nach Anspruch 13, dadurch gekennzeichnet, dass man die Dehydrierung durch einfaches Erhitzen in sauerstoffhaltiger Atmosphäre, gegebenenfalls in Gegenwart eines Dehydrierungskatalysators durchführt.

15. Verfahren zur Herstellung einer Verbindung der Formel I in Anspruch 1, bei der $R^1$, $R^2$, $R^3$, $R^5$, $R^6$, $R^7$ und n die gleiche Bedeutung wie in Anspruch 1 haben und der Heterocyclus

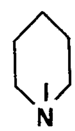

den Pyridinring

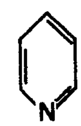

und $R^4$ eine Cyanoalkylgruppe bedeutet, dadurch gekennzeichnet, dass man Natriumcyanid mit einem Chloralkylpyridin der Formel

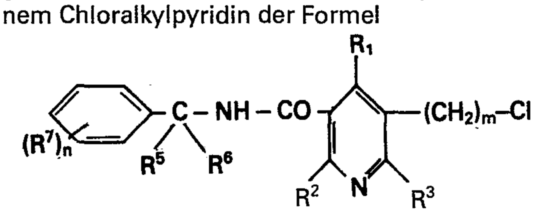

in der $R^1$, $R^2$, $R^3$, $R^5$, $R^6$, $R^7$ und n die gleiche Bedeutung haben, wie in Anspruch 1 und n die gleiche Zahl von 1 bis 4 ist, umsetzt.

16. Verfahren zur Herstellung einer Verbindung der Formel I in Anspruch 1, bei der $R^1$, $R^2$, $R^3$, $R^5$, $R^6$, $R^7$ und n die gleiche Bedeutung wie in Anspruch 1 haben und der Heterocyclus

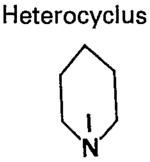

den Pyridinring

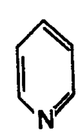

und $R^4$ eine Alkoxyalkylgruppe bedeutet, dadurch gekennzeichnet, dass man Alkalialkanolat mit einem Chloralkylpyridin der Formel

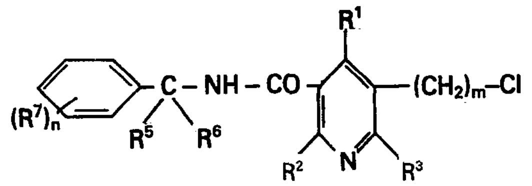

in der $R^1$, $R^2$, $R^3$, $R^5$, $R^6$, $R^7$ und n die gleiche Bedeutung wie in Anspruch 1 haben und n die gleiche Zahl von 1 bis 4 ist, umsetzt.

17. Verfahren zur selektiven Unkrautvertilgung in Anpflanzungen, vor allem von Baumwolle, Sonnenblumen, Weizen oder Gerste, dadurch gekennzeichnet, dass man auf die Pflanzen und/oder auf den Boden des Bereiches, in dem das Unkraut vertilgt werden soll, eine wirksame Menge eines Mittels nach einem der Ansprüche 1 bis 8 aufbringt.

18. Verfahren nach Anspruch 17, dadurch gekennzeichnet, dass man 0,1 bis 10 kg/ha Wirkstoff aufbringt.

19. Verfahren nach Anspruch 17 oder 18, dadurch gekennzeichnet, dass die Behandlung vor dem Auflaufen der Nutzpflanzen oder vor der Aussaat der Nutzpflanzen mit Einarbeiten (in den Boden) vorgenommen wird.

**Claims for the Contracting States**
**BE CH DE FR GB IT LI LU NL SE**

1. Compounds which are in particular useful as herbicides, characterised in that they have the formula

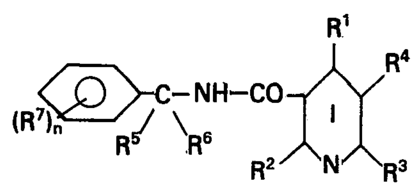

(I)

in which:

$R^1$, $R^2$ and $R^3$, which may be identical or different, represent a hydrogen atom or an alkyl radical having at most 6 carbon atoms or an alkoxy radical having at most 6 carbon atoms or an alkoxyalkyl radical having from 2 to 8 carbon atoms,

$R^4$ represents the carboxyl radical, optionally in the form of a salt or ester, or the cyano radical, or a cyanoalkyl radical having at most 6 carbon atoms or an alkoxyalkyl radical having from 2 to 8 carbon atoms,

$R^5$ and $R^6$ represent a hydrogen atom or an alkyl radical having at most 6 carbon atoms or together constitute a single divalent alkylene radical having from 2 to 5 carbon atoms, and one of these two radicals $R^5$ and $R^6$ can also represent a cyano radical,

$R^7$ represents a halogen atom or an alkyl radical having at most 6 carbon atoms or an alkoxy radical having at most 6 carbon atoms or an alkenyl radical having at most 6 carbon atoms or an alkenyloxy radical having at most 6 carbon atoms (these various alkyl, alkoxy, alkenyl and alkenyloxy radicals being optionally halogenated) or a nitro or cyano radical, or an amino radical, or two $R^7$ together form an alkylenedioxy radical containing from 1 to 4 carbon atoms,

n is a positive integer or zero and is at most equal to 5 and

the heterocyclic

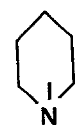

represents a 6-sided unsaturated nitrogen-containing heterocyclic ring containing 2 or 3 unsaturated bonds.

2. Compounds according to claim 1, charcaterised in that

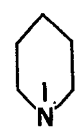

represents the dihydropyridine ring

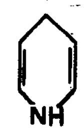

3. Compounds according to claim 1, charcaterised in that

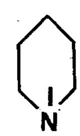

represents the pyridine ring

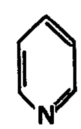

4. Compounds according to one of claims 1 to 3, characterised in that $R^4$ represents a $-COOR^8$ radical, $R^8$ being an alkyl radical containing at most 6 carbon atoms, or $R^4$ represents an alkoxyalkyl radical or a cyanoalkyl radical.

5. Compounds according to one of claims 1 to 4, characterised in that

$R^1$, is a hydrogen atom,

$R^2$ and $R^3$ are the methyl or methoxy or methoxymethyl radical,

$R^4$ is $COOR^8$, $R^8$ having from 1 to 4 carbon atoms, or $R^4$ is a cyanomethyl or alkoxymethyl radical having at most 6 carbon atoms,

$R^5$ and $R^6$ are a hydrogen atom or one of them can be the methyl radical, or $R^5$ and $R^6$ together form an ethylene chain,

$R^7$ is a chlorine or fluorine atom or an alkyl radical which has from 1 to 4 carbon atoms and is optionally halogenated, or an alkoxy radical having from 1 to 4 carbon atoms and

n is 0, 1 or 2.

6. Compounds according to one of claims 1 to 5, characterised in that they are in the form of an optical isomer of the same configuration as (1)-α-methyl-benzylamine.

7. Process for the preparation of compounds according to one of claims 2 and 4 to 6, characterised in that an aldehyde of the formula $R^1$–CHO is reacted with an aminoethylene derivative of the formula (IV) and with a ketone-amide of the formula (V)

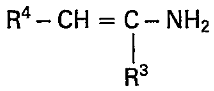

(IV)

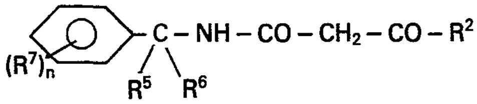

(V)

in which $R^1$, $R^2$, $R^3$, $R^4$, $R^5$, $R^6$, $R^7$ and n have the meanings given in claims 2 and 4 to 6.

8. Process according to claim 7, characterised in that the reaction is carried out at between 15 and 100°C in the presence of an inert solvent.

9. Process according to one of claims 7 or 8, charcterised in that the concentration of the reactants is greater than 5% by weight and that their relative proportions differ by less than 10 mole % from the stoichiometric proportions.

10. Process for the preparation of compounds according to one of claims 3 to 6, characterised in that a compound according to claim 2 is dehydrogenated.

11. Process according to claim 10, characterised in that the dehydrogenation is carried out by means of an oxidising agent such as nitric or nitrous or chromic acid, iodine or sulphur.

12. Process according to claim 10, characterised in that the dehydrogenation is carried out by simple heating in an atmosphere containing oxygen, optionally in the presence of a dehydrogenation catalyst.

13. Process for the preparation of a compound according to claim 1, for which

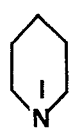

represents the pyridine ring and $R_4$ represents a cyanoalkyl radical, characterised in that sodium cyanide is reacted with the chloroalkyl-pyridine of the formula

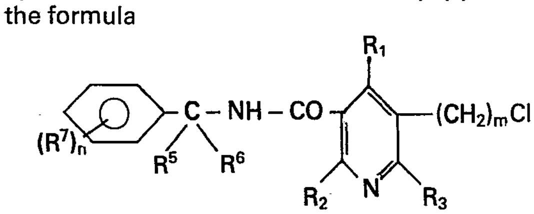

in which $R_1$, $R_2$, $R_3$, $R_5$, $R_6$, $R_7$ and n have the same meaning as in claim 1 and m represents an integer from 1 to 4.

14. Process for the preparation of a compound according to claim 1, in which

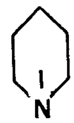

represents the pyridine ring and $R_4$ represents an alkoxyalkyl radical, characterised in that it consists of reacting an alkali metal alkanolate with the chloroalkyl-pyridine of the formula

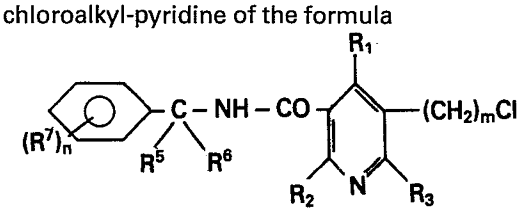

in which $R_1$, $R_2$, $R_3$, $R_5$, $R_6$, $R_7$ and n have the same meaning as in claim 1 and m represents an integer from 1 to 4.

15. Herbicidal composition, characterised in that it contains at least one compound according to claims 1 to 6 as the active material.

16. Composition according to claim 15, characterised in that it contains from 0.05 to 95% by weight of active material.

17. Composition according to one of claims 15 to 16, characterised in that it moreover contains 1 to 94.95% of a solid or liquid support and, optionally 0.1 to 20% of one or more surfactants.

18. Process for selective weeding of crops, especially of cotton, sunflower, wheat or barley, characterised in that an effective quantity of least one compound according to one of claims 1 to 6 is applied ot the plants and/or to the soil of the zone which is to be weeded.

19. Process according to claim 18, characterised in that between 0.1 to 10 kg/ha of the active material is applied.

20. Process according to one of claims 18 or 19, characterised in that the application is effected before emergence of the crops or before sowing the crops, with incorporation.

**Claims for the Contracting State AT**

1. Herbicidal compositon, characterised in that it contains, as the active material, at least one compounds according to the following formula (I):

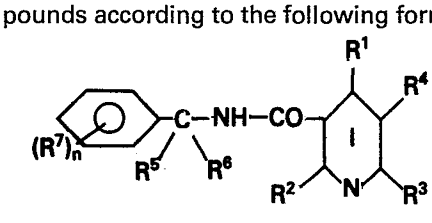

(I)

in which:

$R^1$, $R^2$ and $R^3$, which may be identical or different, represent a hydrogen atom or an alkyl radical having at most 6 carbon atoms or an alkoxy radical having at most 6 carbon atoms or an alkoxyalkyl radical having from 2 to 8 carbon atoms,

$R^4$ represents the carboxyl radical, optionally in the form of a salt or ester, or the cyano radical, or a cyanoalkyl radical having at most 6

carbon atoms or an alkoxyalkyl radical having from 2 to 8 carbon atoms,

$R^5$ and $R^6$ represent a hydrogen atom or an alkyl radical having at most 6 carbon atoms or together constitute a single divalent alkylene radical having from 2 to 5 carbon atoms, and one of these two radicals $R^5$ and $R^6$ can also represent a cyano radical,

$R^7$ represents a halogen atom or an alkyl radical having at most 6 carbon atoms or an alkoxy radical having at most 6 carbon atoms or an alkenyl radical having at most 6 carbon atoms or an alkenyloxy radical having at most 6 carbon atoms (these various alkyl, alkoxy, alkenyl and alkenyloxy radicals being optionally halogenated) or a nitro or cyano radical, or an amino radical, or two $R^7$ together form an alkylenedioxy radical containing from 1 to 4 carbon atoms,

n is a positive integer or zero and is at most equal to 5 and

the heterocyclic

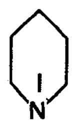

represents a 6-sided unsaturated nitrogen-containing heterocyclic ring containing 2 or 3 unsaturated bonds.

2. Composition according to claim 1, charcaterised in that in formula (I) the heterocyclic ring

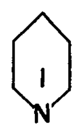

represents the dihydropyridine ring

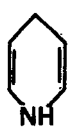

3. Composition according to claim 1, charcaterised in that in formula (I) the heterocyclic ring

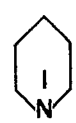

represents the pyridine ring

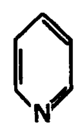

4. Composition according to one of claims 1 to 3, characterised in that in formula (I) $R^4$ represents a $-COOR^8$ radical, $R^8$ being an alkyl radical having at most 6 carbon atoms, or $R^4$ represents a cyanoalkyl radical or alkoxyalkyl radical.

5. Composition according to one of claims 1 to 4, characterised in that in formula (I),

$R^1$, is a hydrogen atom,

$R^2$ and $R^3$ are the methyl or methoxy or methoxymethyl radical,

$R^4$ is $COOR^8$, $R^8$ having from 1 to 4 carbon atoms, or $R^4$ is a cyanomethyl or alkoxymethyl radical having at most 6 carbon atoms,

$R^5$ and $R^6$ are a hydrogen atom or one of them can be the methyl radical, or $R^5$ and $R^6$ together from an ethylene chain,

$R^7$ is a chlorine or fluorine atom or an alkyl radical which has from 1 to 4 carbon atoms and is optionally halogenated, or an alkoxy radical having from 1 to 4 carbon atoms and

n is 0, 1 or 2.

6. Composition according to one of claims 1 to 5, characterised in that the compound of the formula (I) is in the form of an optical isomer of the same configuration as (1)-α-methyl-benzylamine.

7. Composition according to one of claims 1 to 6, characterised in that it contains from 0.05 to 95% by weight of active material.

8. Composition according to claim 7, characterised in that it moreover contains 1 to 94.95% of a solid or liquid carrier and, optionally 0.1 to 20% of one or more surfactants.

9. Process for the preparation of a compound according to formula (I) given in claim 1, in which $R^1$, $R^2$, $R^3$, $R^4$, $R^5$, $R^6$, $R^7$ and n have the same meaning as in claim 1 and the heterocyclic ring

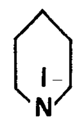

represents the dihydropyridine ring

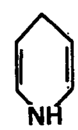

characterised in that an aldehyde of the formula $R^1$–CHO is reacted with an aminoethylene derivative of the formula (IV) and with a ketone-amide of the formula (V)

$$R^4-CH=\underset{\textstyle R^3}{\underset{|}{C}}-NH_2 \qquad (IV)$$

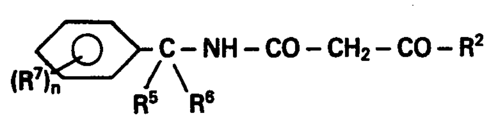 (V)

in which $R^1$, $R^2$, $R^3$, $R^4$, $R^5$, $R^6$, $R^7$ and n have the meanings given earlier.

10. Process according to claim 9, characterised in that the reaction is carried out at between 15 and 100°C in the presence of an inert solvent.

11. Process according to one of claims 9 or 10, characterised in that the concentration of the reactants is greater than 5% by weight and that their relative proportions differ by less than 10 mole % from the stoichiometric proportions.

12. Process for the preparation of a compound according to formula (I) given in claim 1, in which $R^1$, $R^2$, $R^3$, $R^4$, $R^5$, $R^6$, $R^7$ and n have the same meaning as in claim 1 and the heterocyclic ring

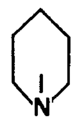

represents the pyridine ring

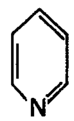

characterised in that the product obtained according to claim 9 is dehydrogenated.

13. Process according to claim 12, characterised in that the dehydrogenation is carried out by means of an oxidising agent such as nitric or nitrous or chromic acid, iodine or sulphur.

14. Process according to claim 13, characterised in that the dehydrogenation is carried out by simple heating in an atmosphere containing oxygen, optionally in the presence of a dehydrogenation catalyst.

15. Process for the preparation of a compound according to formula (I) given in claim 1, in which $R^1$, $R^2$, $R^3$, $R^5$, $R^6$, $R^7$ and n have the same meaning as in claim 1, the heterocyclic ring

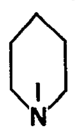

represents the pyridine

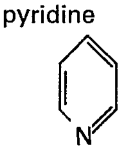

and $R_4$ represents a cyanoalkyl radical, characterised in that it consists in reacting sodium cyanide with the chloroalkyl-pyridine of the formula

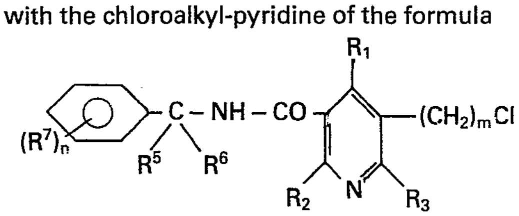

in which $R^1$, $R^2$, $R^3$, $R^5$, $R^6$, $R^7$ and n have the same meaning as in claim 1 and m represents an integer from 1 to 4.

16. Process for the preparation of a compound according to formula (I) given in claim 1, in which $R^1$, $R^2$, $R^3$, $R^5$, $R^6$, $R^7$ and n have the same meaning as in claim 1, the heterocyclic ring

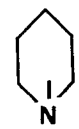

represents the pyridine ring

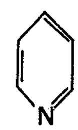

and $R_4$ represents an alkoxyalkyl radical, characterised in that it consists in reacting an alkali metal alkanolate with the chloroalkyl-pyridine of the formula

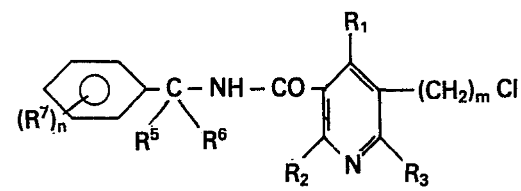

in which $R_1$, $R_2$, $R_3$, $R_5$, $R_6$, $R_7$ and n have the same meaning as in formula (I) of claim 1 and m represents an integer from 1 to 4.

17. Process for the selective weeding of crops, especially of cotton, sunflower, wheat or barley, characterised in that an effective quantity of a composition according to one of claims 1 to 8 is applied to the plants and/or to the soil of the zone which is to be weeded.

18. Process according to claim 17, characterised in that between 0.1 to 10 kg/ha of the active material is applied.

19. Process according to one of claims 17 or 18, characterised in that the application is effected before emergence of the crops or before sowing the crops, with incorporation.